# EUROPEAN PATENT APPLICATION

(11) **EP 4 108 240 A2**
(43) Date of publication of application: **28.12.2022**
(21) Application number: 22180622.7
(22) Date of filing: 23.06.2022
(51) Int. Cl.: A61K 31/4184, A61K 31/4439, A61K 31/444, A61K 31/517, A61K 31/675, A61P 25/28, A61P 29/00, A61P 43/00

(54) **BENZIMIDAZOLE DERIVATIVES FOR TREATMENT AND/OR PREVENTION OF DISEASES AND DISORDERS MEDIATED BY NLRP3**

(30) Priority: 23.06.2021 US 202163213943 P
(71) Applicant: Yoda Pharmaceuticals Inc., KY1-1208 Grand Cayman (KY)
(72) Inventor: TSENG, YUFENG JANE, STAFFORD, 22556-8616 (US)
(74) Representative: Lavoix

(57) **Abstract**

The present disclosure relates to a method for prevention and/or treatment of a disease or disorder mediated by NLRP3 in a subject, comprising administering an effective amount of a compound of Formula (I) or a pharmaceutically acceptable salt, solvate or prodrug as disclosed herein

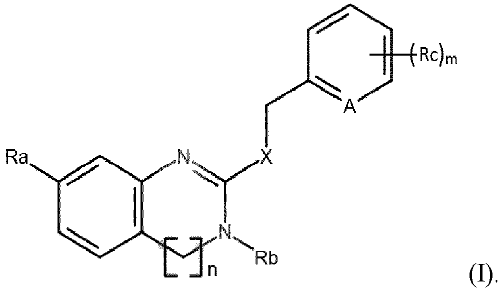

## Description

### Field of the Invention

The present disclosure relates to a method for treating and/or preventing diseases and disorders mediated by NOD-like receptor protein 3 (NLRP3). Particularly, the method uses benzimidazole derivatives to treat and/or prevent diseases and disorders mediated by NLRP3.

### Background of the Invention

The NOD-like receptor protein 3 (NLRP3) is a protein-coding gene: the protein belongs to the family of nucleotide-binding and oligomerization domain-like receptors (NLRs) and is also known as "pyrin domain-containing protein 3." This gene encodes a protein containing a pyrin domain, a nucleotide-binding site domain (NBD), and a leucine-rich repeat (LRR) motif. In response to sterile inflammatory danger signals, NLRP3 interacts with an adapter protein, apoptosis-associated speck-like protein (ASC) and procaspase-1 to form the NLRP3 inflammasome. NLRP3 inflammasome activation then leads to the release of the inflammatory cytokines IL-1β and IL-18.

Dysregulation of inflammasome activation often contributes to human diseases, including inflammatory bowel disease, gout, type II diabetes, cardiovascular disease, Alzheimer's disease and sepsis, the often fatal response to systemic infection, as well as rare genetic diseases caused by mutations of NLRP3 and Pyrin inflammasomes. Gain-of-function mutations in the NLRP3 gene lead to cryopyrin-associated periodic syndromes including familial cold urticaria syndrome, Muckle-Wells syndrome, and chronic infantile neurological cutaneous and articular syndrome that is also known as neonatal onset multisystemic inflammatory disease.

Therefore, there is a need to develop candidate having NLRP3 inflammasome inhibitory effect to provide new and/or alternative treatments for diseases/disorders mediated by NLRP3.

### Summary of the Invention

The present disclosure provides a method for prevention and/or treatment of a disease or disorder mediated by NLRP3 in a subject, comprising administering an effective amount of a benzimidazole derivative as represented by Formula (I) described herein or a pharmaceutically acceptable salt, solvate or prodrug as an active ingredient to the subject.

In some embodiments, the disease or disorder is a neurological disorder, inflammasome-related diseases or disorders, immune diseases, inflammatory diseases, auto-immune diseases, or auto-inflammatory diseases, liver related diseases or disorders, inflammatory arthritis related disorders, kidney related diseases, neuroinflammation-related diseases, cardiovascular or metabolic diseases or disorders, inflammatory skin diseases, wound healing and scar formation, asthma, sarcoidosis, age- related macular degeneration, or cancer related diseases or disorders. Examples of the disease or disorder include, but are not limited to, autoinflammatory fever syndromes, chronic liver disease, viral hepatitis, non alcoholic steatohepatitis (NASH), alcoholic steatohepatitis, alcoholic liver disease, gout, pseudogout (chondrocalcinosis), osteoarthritis, rheumatoid arthritis, arthropathy, hyperoxaluria, lupus nephritis, hypertensive nephropathy, hemodialysis related inflammation, multiple sclerosis, brain infection, acute injury, neurodegenerative diseases, Alzheimer's disease (AD), cardiovascular risk reduction (CvRR), hypertension, atherosclerosis, type I and type II diabetes and related complications, peripheral artery disease (PAD), acute heart failure, hidradenitis suppurativa, acne, colon cancer, lung cancer, myeloproliferative neoplasms, leukemias, myelodysplastic syndromes (MDS), myelofibrosis, multiple system atrophy (MSA), or dementia with Lewy bodies, symptom domains of schizophrenia and schizoaffective disorder (include negative, cognitive, depressive, positive and general psychopathology symptom domains), depression, Tourette Syndrome, Post-traumatic stress disorder (PTSD), Obsessive-compulsive disorder (OCD), analgesics, loss of memory and/or cognition associated with neurodegenerative diseases or loss of neuronal function characteristic of neurodegenerative diseases, mild cognitive impairment (MCI), Alzheimer's disease, Parkinson's disease, schizophrenia, pain, ataxia, convulsion, anxiety, traumatic brain injury (TBI), amyotrophic lateral sclerosis (ALS), multiple sclerosis (MS), fragile X syndrome (FXS), autism, or attention-deficit/hyperactivity disorder (ADHD).

Examples of benzimidazole derivative as represented by Formula (I) includes, but are not limited to, those illustrated in the disclosures thereafter.

### Brief Description of the Drawing

Figure 1 shows results of western blot in human THP-1 cells.
Figure 2A show ELISA results in LPS- and ATP-treated cells.
Figure 2B shows ELISA results in LPS- and MSU-treated cells.
Figure 3 shows the treatment effect of RS-D7 on neuronal alpha-synucleinopathy related locomotion activity deficits in TgM83 mouse model of neurodegenerative diseases.
Figure 4 shows the treatment effect of RS-D7 on neuronal alpha-synucleinopathy related motor coordination deficits in TgM83 mouse model of neurodegenerative diseases.
Figure 5A shows the treatment effect of RS-D7 on inflammation cytokines IL-1β in the cerebellum of TgM83 mouse model of neurodegenerative diseases.
Figure 5B shows the treatment effect of RS-D7 on inflammation cytokine IL-6 in the cerebellum of TgM83 mouse model of neurodegenerative diseases.
Figure 5C shows the treatment effect of RS-D7 on inflammation cytokine TNF-α in the cerebellum of TgM83 mouse model of neurodegenerative diseases.

### Detailed Description of the Invention

As used herein and in the appended claims, the singular forms "a," "and," and "the" include plural referents unless the context clearly dictates otherwise. When ranges are used herein for physical properties, such as molecular weight, or chemical properties, such as chemical formulae, all combinations and subcombinations of ranges and specific embodiments therein are intended to be included. The term "or" refers to "and/or" unless explicitly indicated to refer to alternatives only or unless the alternatives are mutually exclusive. The term "about" when referring to a number or a numerical range means that the number or numerical range referred to is an approximation within experimental variability (or within statistical experimental error). The term "comprising" (and related terms such as "comprise" or "comprises" or "having" or "including") is not intended to exclude that in other certain embodiments, for example, an embodiment of any composition of matter, composition, method, or process, or the like, described herein, may "consist of" or "consist essentially of" the described features.

### Definitions

"Alkyl" refers to a straight or branched hydrocarbon chain radical consisting solely of carbon and hydrogen atoms, containing no unsaturation, having from one to fifteen carbon atoms (e.g., C₁-C₁₅ alkyl). In certain embodiments, an alkyl comprises one to thirteen carbon atoms (e.g., C₁-C₁₀ alkyl). In certain embodiments, an alkyl comprises one to eight carbon atoms (e.g., C₁-C₈ alkyl). In other embodiments, an alkyl comprises one to five carbon atoms (e.g., C₁-C₆ alkyl). In other embodiments, an alkyl comprises one to four carbon atoms (e.g., C₁-C₄ alkyl). In other embodiments, an alkyl comprises one to three carbon atoms (e.g., C₁-C₃ alkyl). In other embodiments, an alkyl comprises one to two carbon atoms (e.g., C₁-C₂ alkyl). In other embodiments, an alkyl comprises one carbon atom (e.g., C₁ alkyl). In other embodiments, an alkyl comprises five to fifteen carbon atoms (e.g., C₅-C₁₅ alkyl). In other embodiments, an alkyl comprises five to eight carbon atoms (e.g., C₅-C₈ alkyl). In other embodiments, an alkyl comprises two to five carbon atoms (e.g., C₂-C₅ alkyl). In other embodiments, an alkyl comprises three to five carbon atoms (e.g., C₃-C₅ alkyl). In other embodiments, the alkyl group is selected from methyl, ethyl, 1-propyl (n-propyl), 1-methylethyl (iso-propyl), 1-butyl (n-butyl), 1-methylpropyl (sec-butyl), 2-methylpropyl (iso-butyl), 1,1-dimethylethyl (tert-butyl), 1-pentyl (n-pentyl). The alkyl is attached to the rest of the molecule by a single bond. Unless specifically stated otherwise in the specification, an alkyl group is optionally substituted by one or more of substituents.

"Alkoxy" refers to a radical bonded through an oxygen atom of the formula -O-alkyl, where alkyl is an alkyl chain as defined above.

"Alkenyl" refers to a straight or branched hydrocarbon chain radical group consisting solely of carbon and hydrogen atoms, containing at least one carbon-carbon double bond, and having from two to twelve carbon atoms. In certain embodiments, an alkenyl comprises two to eight carbon atoms. In other embodiments, an alkenyl comprises two to four carbon atoms. The alkenyl is attached to the rest of the molecule by a single bond, for example, ethenyl (i.e., vinyl), prop-1-enyl (i.e., allyl), but-1-enyl, pent-1-enyl, penta-1,4-dienyl, and the like. Unless specifically stated otherwise in the specification, an alkenyl group is optionally substituted by one or more of substituents.

"Alkynyl" refers to a straight or branched hydrocarbon chain radical group consisting solely of carbon and hydrogen atoms, containing at least one carbon-carbon triple bond, having from two to twelve carbon atoms. In certain embodiments, an alkynyl comprises two to eight carbon atoms. In other embodiments, an alkynyl has two to four carbon atoms. The alkynyl is attached to the rest of the molecule by a single bond, for example, ethynyl, propynyl, butynyl, pentynyl, hexynyl, and the like. Unless specifically stated otherwise in the specification, an alkynyl group is optionally substituted by one or more of substituents.

"Aryl" refers to a radical derived from an aromatic monocyclic or multicyclic hydrocarbon ring system by removing a hydrogen atom from a ring carbon atom. The aromatic monocyclic or multicyclic hydrocarbon ring system contains only hydrogen and carbon from five to eighteen carbon atoms, where at least one of the rings in the ring system is fully unsaturated, i.e., it contains a cyclic, delocalized (4n+2) pi-electron system in accordance with the Huckel theory. The ring system from which aryl groups are derived include, but are not limited to, groups such as benzene, fluorene, indane, indene, tetralin and naphthalene. Unless stated otherwise specifically in the specification, the term "aryl" or the prefix "ar-" (such as in "aralkyl") is meant to include aryl radicals optionally substituted by one or more substituents independently selected from alkyl, alkenyl, alkynyl, halo, fluoroalkyl, cyano, nitro, optionally substituted aryl, optionally substituted aralkyl, optionally substituted aralkenyl, optionally substituted aralkynyl, optionally substituted carbocyclyl, optionally substituted carbocyclylalkyl, optionally substituted heterocyclyl, optionally substituted heterocyclylalkyl, optionally substituted heteroaryl, optionally substituted heteroarylalkyl, -R^{b}--OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R^{b}-C(O)OR^{a}, -R^{b}-C(O)N(R^{a})₂, -R^{b}-O-R-C(O)N(R^{a})₂, -R^{b}--N(R^{a})C(O)OR^{a}, - R^{b}-N(R^{a}) C(O)R^{a}, -R^{b}-N(R^{a})S(O)ₜR^{a} (where t is 1 or 2), -R^{b}-S(O)ₜOR^{a} (where t is 1 or 2), -R^{b}-S(O).subₜR^{a} (where t is 1 or 2) and -R^{b}-S(O)ₜN(R^{a})₂ (where t is 1 or 2), where each R^{a} is independently hydrogen, alkyl, fluoroalkyl, cycloalkyl, cycloalkylalkyl, aryl (optionally substituted with one or more halo groups), aralkyl, heterocyclyl, heterocyclylalkyl, heteroaryl or heteroarylalkyl, each R.sup.b is independently a direct bond or a straight or branched alkylene or alkenylene chain, and R.sup.c is a straight or branched alkylene or alkenylene chain, and where each of the above substituents is unsubstituted unless otherwise indicated.

"Heteroaryl" refers to a radical derived from a 3- to 18-membered aromatic ring radical that comprises two to seventeen carbon atoms and from one to six heteroatoms selected from nitrogen, oxygen and sulfur. As used herein, the heteroaryl radical may be a monocyclic, bicyclic, tricyclic or tetracyclic ring system, wherein at least one of the rings in the ring system is fully unsaturated, i.e., it contains a cyclic, delocalized (4n+2) pi-electron system in accordance with the Huckel theory. Heteroaryl includes fused or bridged ring systems. The heteroatom(s) in the heteroaryl radical is optionally oxidized. One or more nitrogen atoms, if present, are optionally quaternized. The heteroaryl is attached to the rest of the molecule through any atom of the ring(s). Examples of heteroaryls include, but are not limited to, azepinyl, acridinyl, benzimidazolyl, benzindolyl, 1,3-benzodioxolyl, benzofuranyl, benzooxazolyl, benzo[d]thiazolyl, benzothiadiazolyl, benzo[b][1,4]dioxepinyl, benzo[b][1,4]oxazinyl, 1,4-benzodioxanyl, benzonaphthofuranyl, benzoxazolyl, benzodioxolyl, benzodioxinyl, benzopyranyl, benzopyranonyl, benzofuranyl, benzofuranonyl, benzothienyl (benzothiophenyl), benzothieno[3,2-d]pyrimidinyl, benzotriazolyl, benzo[4,6]imidazo[1,2-a]pyridinyl, carbazolyl, cinnolinyl, cyclopenta[d]pyrimidinyl, 6,7-dihydro-5H-cyclopenta[4,5]thieno[2,3-d]pyrimidinyl, 5,6-dihydrobenzo[h]quinazolinyl, 5,6-dihydrobenzo[h]cinnolinyl, 6,7-dihydro-5H-benzo[6,7]cyclohepta[1,2-c]pyridazinyl, dibenzofuranyl, dibenzothiophenyl, furanyl, furanonyl, furo [3,2-c]pyridinyl, 5,6,7,8,9,10-hexahydrocycloocta[d]pyrimidinyl, 5,6,7,8,9,10-hexahydrocycloocta[d]pyridazinyl, 5,6,7,8,9,10-hexahydrocycloocta[d]pyridinyl, isothiazolyl, imidazolyl, indazolyl, indolyl, indazolyl, isoindolyl, indolinyl, isoindolinyl, isoquinolyl, indolizinyl, isoxazolyl, 5,8-methano-5,6,7,8-tetrahydroquinazolinyl, naphthyridinyl, 1,6-naphthyridinonyl, oxadiazolyl, 2-oxoazepinyl, oxazolyl, oxiranyl, 5,6,6a,7,8,9,10,10a-octahydrobenzo[h]quinazolinyl, 1-phenyl-1H-pyrrolyl, phenazinyl, phenothiazinyl, phenoxazinyl, phthalazinyl, pteridinyl, purinyl, pyrrolyl, pyrazolyl, pyrazolo[3,4-d]pyrimidinyl, pyridinyl, pyrido[3,2-d]pyrimidinyl, pyrido[3,4-d]pyrimidinyl, pyrazinyl, pyrimidinyl, pyridazinyl, pyrrolyl, quinazolinyl, quinoxalinyl, quinolinyl, isoquinolinyl, tetrahydroquinolinyl, 5,6,7,8-tetrahydroquinazolinyl, 5,6,7,8-tetrahydrobenzo[4,5]thieno[2,3-d]pyrimidinyl, 6,7,8,9-tetrahydro-5H-cyclohepta[4,5]thieno[2,3-d]pyrimidinyl, 5,6,7,8-tetrahydropyrido[4,5-c]pyridazinyl, thiazolyl, thiadiazolyl, triazolyl, tetrazolyl, triazinyl, thieno[2,3-d]pyrimidinyl, thieno[3,2-d]pyrimidinyl, thieno[2,3-c]pyridinyl, and thiophenyl (i.e. thienyl). Unless stated otherwise specifically in the specification, the term "heteroaryl" is meant to include heteroaryl radicals as defined above which are optionally substituted by one or more substituents selected from alkyl, alkenyl, alkynyl, halo, fluoroalkyl, haloalkenyl, haloalkynyl, oxo, thioxo, cyano, nitro, optionally substituted aryl, optionally substituted aralkyl, optionally substituted aralkenyl, optionally substituted aralkynyl, optionally substituted carbocyclyl, optionally substituted carbocyclylalkyl, optionally substituted heterocyclyl, optionally substituted heterocyclylalkyl, optionally substituted heteroaryl, optionally substituted heteroarylalkyl, -R^{b}--OR^{a}, -R^{b}-OC(O)-R^{a}, -R^{b}-OC(O)-OR^{a}, -R^{b}-OC(O)-N(R^{a})₂, -R^{b}--N(R^{a})₂, -R^{b}-C(O)R^{a}, -R^{b}-C(O)OR^{a}, -R^{b}-C(O)N(R^{a})₂, -R^{b}-O-R-C(O)N(R^{a})₂, - R^{b}--N(R^{a})C(O)OR^{a}, -R^{b}-N(R^{a}) C(O)R^{a}, -R^{b}-N(R^{a})S(O)ₜR^{a} (where t is 1 or 2), -R^{b}-S(O)ₜOR^{a} (where t is 1 or 2), -R^{b}--S(O).subₜR^{a} (where t is 1 or 2) and -R^{b}-S(O)ₜN(R^{a})₂ (where t is 1 or 2), where each R^{a} is independently hydrogen, alkyl, fluoroalkyl, cycloalkyl, cycloalkylalkyl, aryl, aralkyl, heterocyclyl, heterocyclylalkyl, heteroaryl or heteroarylalkyl, each R.sup.b is independently a direct bond or a straight or branched alkylene or alkenylene chain, and R.sup.c is a straight or branched alkylene or alkenylene chain, and where each of the above substituents is unsubstituted unless otherwise indicated.

The term "pharmaceutically acceptable salt" refers to salts prepared from pharmaceutically acceptable non-toxic bases or acids including inorganic or organic bases and inorganic or organic acids. Salts of basic compounds encompassed within the term "pharmaceutically acceptable salt" refer to non-toxic salts of the compounds of the present disclosure which are generally prepared by reacting the free base with a suitable organic or inorganic acid. Representative salts of basic compounds of the present disclosure include, but are not limited to, the following: acetate, ascorbate, adipate, alginate, aspirate, benzenesulfonate, benzoate, bicarbonate, bisulfate, bitartrate, borate, bromide, butyrate, camphorate, camphorsulfonate, camsylate, carbonate, chloride, clavulanate, citrate, cyclopentane propionate, diethylacetic, digluconate, dihydrochloride, dodecylsulfanate, edetate, edisylate, estolate, esylate, ethanesulfonate, formic, fumarate, gluceptate, glucoheptanoate, gluconate, glutamate, glycerophosphate, glycollylarsanilate, hemisulfate, heptanoate, hexanoate, hexylresorcinate, hydrabamine, hydrobromide, hydrochloride, 2-hydroxyethanesulfonate, hydroxynaphthoate, iodide, isonicotinic, isothionate, lactate, lactobionate, laurate, malate, maleate, mandelate, mesylate, methylbromide, methylnitrate, methylsulfate, methanesulfonate, mucate, 2-naphthalenesulfonate, napsylate, nicotinate, nitrate, N-methylglucamine ammonium salt, oleate, oxalate, pamoate (embonate), palmitate, pantothenate, pectinate, persulfate, phosphate/diphosphate, pimelic, phenylpropionic, polygalacturonate, propionate, salicylate, stearate, sulfate, subacetate, succinate, tannate, tartrate, teoclate, thiocyanate, tosylate, triethiodide, trifluoroacetate, undeconate, valerate and the like. Furthermore, where the compounds of the present disclosure carry an acidic moiety, suitable pharmaceutically acceptable salts thereof include, but are not limited to, salts derived from inorganic bases including aluminum, ammonium, calcium, copper, ferric, ferrous, lithium, magnesium, manganic, mangamous, potassium, sodium, zinc, and the like. Also included are the ammonium, calcium, magnesium, potassium, and sodium salts. Salts derived from pharmaceutically acceptable organic non-toxic bases include salts of primary, secondary, and tertiary amines, cyclic amines, dicyclohexyl amines and basic ionexchange resins, such as arginine, betaine, caffeine, choline, N,N-dibenzylethylenediamine, diethylamine, 2-diethylaminoethanol, 2-dimethylaminoethanol, ethanolamine, ethylamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like. Also, included are the basic nitrogen-containing groups that may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others.

The term "pharmaceutical composition" refers to a compound of the present disclosure, or a pharmaceutically acceptable salt thereof, together with at least one pharmaceutically acceptable carrier, in a form suitable for oral or parenteral administration.

The term "pharmaceutically acceptable carrier" refers to a substance useful in the preparation or use of a pharmaceutical composition and includes, for example, suitable diluents, solvents, dispersion media, surfactants, antioxidants, preservatives, isotonic agents, buffering agents, emulsifiers, absorption delaying agents, salts, drug stabilizers, binders, excipients, disintegration agents, lubricants, wetting agents, sweetening agents, flavoring agents, dyes, and combinations thereof, as would be known to those skilled in the art.

The term "subject" includes living organisms such as humans, monkeys, cows, sheep, horses, pigs, cattle, goats, dogs, cats, mice, rats, cultured cells, and transgenic species thereof. In a preferred embodiment, the subject is a human.

The terms "administer," "administering," or "administration," as used herein, refers to implanting, absorbing, ingesting, injecting, inhaling, or otherwise introducing an inventive compound, or a pharmaceutical composition thereof, in or on a subject. The terms "coadministration" and "in combination with" include the administration of two or more therapeutic agents simultaneously, concurrently, separately or sequentially within no specific time limits unless otherwise indicated. In one embodiment, the therapeutic agents are in the same composition or unit dosage form. In other embodiments, the therapeutic agents are in separate compositions or unit dosage forms.

As used herein, the terms "condition," "disease," and "disorder" are used interchangeably.

The term "treat" or "treatment" refers to a method of reducing the effects of a disease or condition. Treatment can also refer to a method of reducing the underlying cause of the disease or condition itself rather than just the symptoms. The treatment can be any reduction from native levels and can be, but is not limited to, the complete ablation of the disease, condition, or the symptoms of the disease or condition.

As used herein, the terms "prevent," "preventing" and "prevention" refer to the prevention of the onset, recurrence or spread of a disease or disorder, or of one or more symptoms thereof. In certain embodiments, the terms refer to the treatment with or administration of a compound or an antibody or dosage form provided herein, with or without one or more other additional active agents, prior to the onset of symptoms, particularly to patients at risk of disease or disorders provided herein. The terms encompass the inhibition or reduction of a symptom of the particular disease. In this regard, the term "prevention" may be interchangeably used with the term "prophylactic treatment."

The term "a therapeutically effective amount" of a compound used in the present disclosure refers to an amount of the compound that will elicit the biological or medical response of a subject, for example, reduction or inhibition of an enzyme or a protein activity, or ameliorate symptoms, alleviate conditions, slow or delay disease progression, or prevent a disease, etc. In one embodiment, the term "a therapeutically effective amount" refers to the amount of the compound used in the present disclosure that, when administered to a subject, is effective to (1) at least partially alleviate, inhibit, prevent and or or ameliorate a condition, or a disorder or a disease (i) mediated by NLRP3, or (ii) associated with NLRP3 activity, or (iii) characterized by activity (normal or abnormal) of NLRP3; or (2) reduce or inhibit the activity of NLRP3; or (3) reduce or inhibit the expression of NLRP3. In another embodiment, the term "a therapeutically effective amount" refers to the amount of the compound used in the present disclosure that, when administered to a cell, or a tissue, or a non-cellular biological material, or a medium, is effective to at least partially reduce or inhibit the activity of NLRP3; or at least partially reduce or inhibit the expression of NLRP3.

The term "inhibit", "inhibition" or "inhibiting" refers to the reduction or suppression of a given condition, symptom, or disorder, or disease, or a significant decrease in the baseline activity of a biological activity or process. Specifically, inhibiting NLRP3 or inhibiting NLRP3 inflammasome pathway comprises reducing the ability of NLRP3 or NLRP3 inflammasome pathway to induce the production of I L-1 b and/or IL-18. This can be achieved by mechanisms including, but not limited to, inactivating, destabilizing, and/or altering distribution of NLRP3.

The term "NLRP3" includes but is not limited to nucleic acids, polynucleotides, oligonucleotides, sense and anti-sense polynucleotide strands, complementary sequences, peptides, polypeptides, proteins, homologous and/or orthologous NLRP molecules, isoforms, precursors, mutants, variants, derivatives, splice variants, alleles, different species, and active fragments thereof.

The term "neurological disorder" refers to any undesirable condition of the central or peripheral nervous system of a mammal. The term "neurological disorder" includes neurodegenerative diseases and neuropsychiatric diseases. Exemplary neurological disorders include Alzheimer's disease, Parkinson's disease, amyotrophic lateral sclerosis, schizophrenia and anxieties, such as general anxiety disorder, MLS (cerebellar ataxia), Huntington's disease, Down syndrome, multi-infarct dementia, status epilecticus, contusive injuries (e.g. spinal cord injury and head injury), viral infection induced neurodegeneration, (e.g. AIDS, encephalopathies), epilepsy, benign forgetfulness, closed head injury, sleep disorders, depression (e.g., bipolar disorder), dementias, movement disorders, psychoses, alcoholism, post-traumatic stress disorder and the like. "Neurological disorder" also includes any undesirable condition associated with the disorder. For instance, a method of treating a neurodegenerative disorder includes methods of treating loss of memory and/or loss of cognition associated with a neurodegenerative disorder. Such method would also include treating or preventing loss of neuronal function characteristic of neurodegenerative disorder.

### Diseases or disorders mediated by NLRP3

Diseases or disorders mediated by NLRP3 are well known in the art. For example, WO 2020021447 A1 discloses diseases or disorders mediated by NLRP3 in detail. NLRP3 inflammasome complexes, including their activations and subsequent release of inflammatory cytokines and downstream signaling events have been linked to neurodegenerative and neurological diseases. Thus, inhibition or modulation of NLRP3 inflammasome-related complexes, cytokines or signaling activities is a promising target for neurodegenerative diseases or neurological diseases treatment. As disclosed in the reference, NLRP3 mutations have been found to be responsible for a set of rare autoinflammatory diseases known as cryopyrin-associated periodic syndrome (CAPS), which are heritable diseases characterized by recurrent fever and inflammation and are comprised of three autoinflammatory disorders that form a clinical continuum. These diseases are familial cold autoinflammatory syndrome (FCAS), Muckle-Wells syndrome (MWS), and chronic infantile cutaneous neurological articular syndrome (CINCA; also called neonatal- onset multisystem inflammatory disease, NOMID), and all have been shown to result from gain-of- function mutations in the NLRP3 gene, which leads to increased secretion of IL-1β. NLRP3 has also been implicated in a number of autoinflammatory diseases, including pyogenic arthritis, pyoderma gangrenosum and acne (PAPA), Sweet's syndrome, chronic nonbacterial osteomyelitis (CNO), and acne vulgaris.

A number of autoimmune diseases have been shown to involve NLRP3 including, in particular, multiple sclerosis, type-1 diabetes, psoriasis, rheumatoid arthritis (RA), Behcet's disease, Schnitzler syndrome, macrophage activation syndrome, systemic lupus erythematosus and its complications such as lupus nephritis, and systemic sclerosis. NLRP3 has also been shown to play a role in a number of lung diseases including chronic obstructive pulmonary disorder (COPD), asthma (including steroid-resistant asthma), asbestosis, and silicosis. NLRP3 has also been suggested to have a role in a number of central nervous system conditions, including Multiple Sclerosis (MS), Parkinson's disease (PD), Alzheimer's disease (AD), dementia, Huntington's disease, cerebral malaria, brain injury from pneumococcal meningitis, intracranial aneurysms, and traumatic brain injury. NRLP3 activity has also been shown to be involved in various metabolic diseases including type 2 diabetes and its organ-specific complications, atherosclerosis, obesity, gout, pseudo-gout, metabolic syndrome, and non-alcoholic steatohepatitis. A role for NLRP3 via IL-1β has also been suggested in atherosclerosis, myocardial infarction, aortic aneurysm and dissection, and other cardiovascular events.

The accumulation of misfolded α-Synuclein aggregated form plays a significant role in neurodegenerative diseases, such as Alzheimer's disease (AD), Parkinson's disease (PD), multiple system atrophy (MSA), and dementia with Lewy bodies. Accumulation evidences indicated that α-Synuclein activated NLRP3 inflammasome, especially in microglia. Moreover, recent studies have also shown that NLRP3 inflammasome was significantly upregulated and correlated with the neurodegenerative processes in AD, PD, MSA and amyotrophic lateral sclerosis (ALS).

In addition, modulators of the inflammasome have demonstrated effect on α-synuclein pathology. Transgenic mice expressing A53T human α-Synuclein in CNS neurons, and M83 expressed behavior deficits and aggregated α-Synuclein inclusions in the cerebellum. TgM83 mice expressing mutant α-Synuclein with age-dependent effect will develop a severe and complex motor, emotional, and cognitive impairment leading to paralysis and death. Aged TgM83 mice also showed α-Synuclein accumulation and nigrostriatal neuron loss in striatum. Studies involving TgM83 mice have suggested therapeutic intervention for synucleinopathies with NLRP3 inflammasome in diseases such as MSA, anxiety, and PD.

Other diseases in which NLRP3 has been shown to be involved include: ocular diseases such as both wet and dry age-related macular degeneration, diabetic retinopathy, non-infectious uveitis and optic nerve damage; liver diseases including non-alcoholic steatohepatitis (NASH) and acute alcoholic hepatitis; inflammatory reactions in the lung and skin including contact hypersensitivity (such as bullous pemphigoid), atopic dermatitis, Hidradenitis suppurativa, and sarcoidosis; inflammatory reactions in the joints; amyotrophic lateral sclerosis; cystic fibrosis; stroke; chronic kidney disease; and inflammatory bowel diseases including ulcerative colitis and Crohn's disease. The NLRP3 inflammasome has been found to be activated in response to oxidative stress. NLRP3 has also been shown to be involved in inflammatory hyperalgesia.

Activation of the NLRP3 inflammasome has been shown to potentiate some pathogenic infections such as influenza and Leishmaniasis.

NLRP3 has also been implicated in the pathogenesis of many cancers. For example, several previous studies have suggested a role for IL-1β in cancer invasiveness, growth and metastasis, and inhibition of IL- 1 beta with canakinumab has been shown to reduce the incidence of lung cancer and total cancer mortality in a randomised, double-blind, placebo-controlled trial. Inhibition of the NLRP3 inflammasome or IL-1β has also been shown to inhibit the proliferation and migration of lung cancer cells in vitro. A role for the NLRP3 inflammasome has been suggested in myelodysplastic syndromes, myelofibrosis and other myeloproliferative neoplasms, and acute myeloid leukemia (AML) and also in the carcinogenesis of various other cancers including glioma, inflammation- induced tumors, multiple myeloma, and squamous cell carcinoma of the head and neck. Activation of the NLRP3 inflammasome has also been shown to mediate chemoresistance of tumor cells to 5-Fluorouracil, and activation of NLRP3 inflammasome in peripheral nerve contributes to chemotherapy-induced neuropathic pain. NLRP3 has also been shown to be required for the efficient control of viruses, bacteria, and fungi.

The activation of NLRP3 leads to cell pyroptosis and this feature plays an important part in the manifestation of clinical disease. Therefore, it is anticipated that inhibitors of NLRP3 will block pyroptosis, as well as the release of pro-inflammatory cytokines (e.g. IL-1β) from the cell.

### Benzimidazole Derivatives For Treating and/or Preventing Diseases or Disorders Mediated by NLRP3

In the present disclosure, the method uses a benzimidazole compound of formula (I): 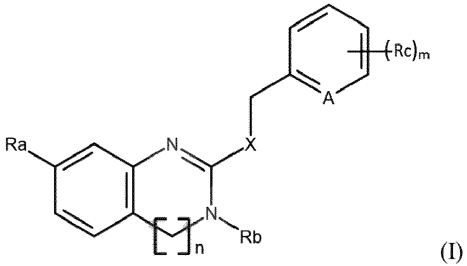 wherein n is 0 or 1,
X is -S-, -S(=O)- or -NRₙ-; wherein
   Rₙ is H or 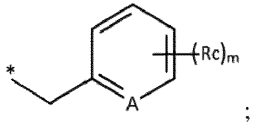
A is -CH, -CR_{c} or N;
Rₐ is -C(=O)ORₐ₁, -ORₐ₂, -O-C(=O)Rₐ₃ or -O-C(=O)-T-ORₐ₄; wherein
   Rₐ₁ is H or linear or branched C₁₋₁₅alkyl;
   Rₐ₂ is H, linear or branched C₁₋₁₅alkyl, phosphonate or diarylphosphonate;
   Rₐ₃ and Rₐ₄ are independently linear or branched C₁₋₁₅alkyl, linear or branched C₂₋₁₅alkenyl, -T-C₃₋₁₀cycloalkyl, -T-NHRₐ₃ₚ, -T-C₃₋₁₀ycloalkenyl, -T-C₆₋₁₀aryl, -T-C₅₋₁₀heteroaryl, -T-NH-C(=O)-O-C₁₋₁₀alkyl, -T-adamantyl or -C₁₋₃alkylene-C₆₋₁₀aryl where the alkylene is substituted with -T-NHRₐ₃ₚ;
   Rₐ₃ₚ is H, fluorenylmethoxycarbonyl (Fmoc) or tert-butoxycarbonyl (Boc);
R_{b} is H, linear or branched C₁₋₁₅alkyl, linear or branched C₂₋₁₅alkenyl, C₁₋₃alkoxy-C₁₋₁₅alkyl-, -T'-C₃₋₁₀cycloalkyl, -T'-C₃₋₁₀cycloalkenyl, -T'-C₆₋₁₀ aryl or -T'-C₅₋₁₀heteroaryl;
R_{c} each is independently halogen, linear or branched C₁₋₁₅alkyl, linear or branched C₁₋₁₅alkoxyl, hydroxyl, or -C₁₋₁₀alkylene-Y-C₆₋₁₀heteroaryl wherein -Y- is -CH₂-, -NH-, -O- or - S-;
symbol ^{∗} represents the bonding position;
m is an integer from 0 to 4;
-T- is absent, C₁₋₃alkylene or C₂₋₃alkenylene;
-T'- is C₁₋₃alkylene or C₂₋₃alkenylene; and
wherein the heteroaryl contains at least one heteroatom, each heteroatom being independently S, N or O;
wherein the alkyl, alkenyl, alkoxy, cycloalkyl, aryl, heteroaryl, alkylene and alkenylene are each independently unsubstituted or substituted with at least one substituent;
wherein the substituent is each independently a halogen, amino, nitro, nitroso, linear or branched C₁₋₁₅ alkyl,or linear or branched C₁₋₁₅ alkoxy or C₃₋₁₀cycloalkyl; and
when R_{b} is H, the tautomers are included,
or a pharmaceutically acceptable salt thereof.

In one embodiment, the compound of formula (I) is a compound of formula (I-a): 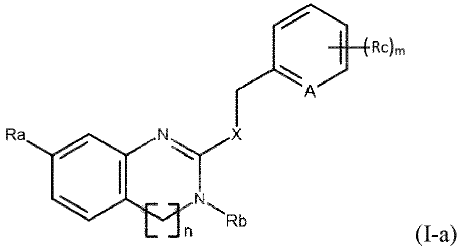 wherein n is 0 or 1,
X is -S-, -S(=O)- or -NRₙ-; wherein
   Rₙ is H or 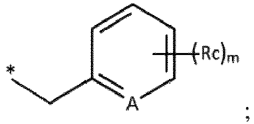
A is -CH, -CR_{c} or N;
Rₐ is -C(=O)ORₐ₁, -ORₐ₂, -O-C(=O)Rₐ₃ or -O-C(=O)-T-ORₐ₄; wherein
   Rₐ₁ is H or linear or branched C₁₋₁₅alkyl;
   Rₐ₂ is H, linear or branched C₁₋₁₅alkyl or diarylphosphonate;
   Rₐ₃ and Rₐ₄ are independently linear or branched C₁₋₁₅alkyl, linear or branched C₂₋₁₅alkenyl, -T-C₃₋₁₀ycloalkyl, -T-NHRₐ₃ₚ, -T-C₃₋₁₀cycloalkenyl, -T-C₆₋₁₀aryl, -T-C₅₋₁₀eteroaryl, -T-NH-C(=O)-O-C₁₋₁₀alkyl or -T-adamantyl;
   Rₐ₃ₚ is H, Fmoc or Boc;
R_{b} is H, linear or branched C₁₋₁₅alkyl, linear or branched C₂₋₁₅alkenyl, C₁₋₃alkoxy-C₁₋₁₅alkyl-, -T'-C₃₋₁₀cycloalkyl, -T'-C₃₋₁₀cycloalkenyl, -T'-C₆₋₁₀ aryl or -T'-C₅₋₁₀heteroaryl;
R_{c} each is independently halogen, linear or branched C₁₋₁₅alkyl, linear or branched C₁₋₁₅alkoxyl, hydroxyl, or -C₁₋₁₀alkylene-Y-C₆₋₁₀heteroaryl wherein -Y- is -CH₂-, -NH-, -O- or - S-;
symbol ^{∗} represents the bonding position;
m is an integer from 0 to 4;
-T- is absent, C₁₋₃alkylene or C₂₋₃alkenylene;
-T'- is C₁₋₃alkylene or C₂₋₃alkenylene; and
wherein the heteroaryl contains at least one heteroatom, each heteroatom being independently S, N or O;
wherein the alkyl, alkenyl, alkoxy, cycloalkyl, aryl, heteroaryl, alkylene and alkenylene are each independently unsubstituted or substituted with at least one substituent;
wherein the substituent is each independently a halogen, amino, nitro, nitroso, linear or branched C₁₋₁₅ alkyl, linear or branched C₁₋₁₅ alkoxy or C₃₋₁₀cycloalkyl and
when R_{b} is H, the tautomers are included,
or a pharmaceutically acceptable salt thereof.

In one embodiment, the compound of formula (I) is a compound of formula (I-b), 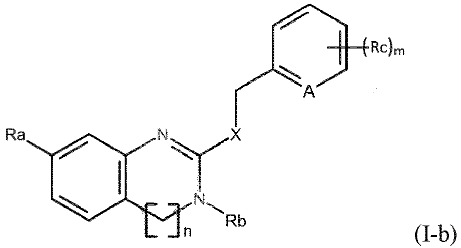 wherein n is 0 or 1,
X is -S-, -S(=O)- or -NRₙ-; Rn is H or 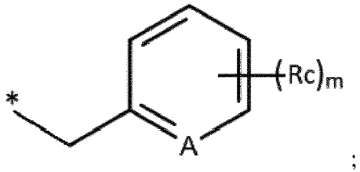
A is -CH, -CR_{c} or N;
Rₐ is -C(=O)ORₐ₁, -ORₐ₂ or -O-C(=O)Rₐ₃; wherein
   Rₐ₁ is H or linear or branched C₁₋₁₅alkyl;
   Rₐ₂ is H, linear or branched C₁₋₁₅alkyl, phosphonate or diarylphosphonate;
   Rₐ₃ is -T-NHRₐ₃ₚ, -T-NH-C(=O)-O-C₁₋₁₀alkyl or -C₁₋₃alkylene-C₆₋₁₀aryl where the alkylene is substituted with -T-NHRₐ₃ₚ;
   Rₐ₃ₚ is H, Fmoc or Boc,
R_{b} is H, linear or branched C₁₋₁₅alkyl, C₁₋₃alkoxy-C₁₋₁₀alkyl-, -T'-C₃₋₁₀ cycloalkyl, -T-C₃₋₁₀cycloalkenyl, -T'-C₆₋₁₀ aryl or -T'-C₅₋₁₀ heteroaryl;
R_{c} each is independently halogen, linear or branched C₁₋₁₅alkyl, linear or branched C₁₋₁₅alkoxyl, hydroxyl or -C₁₋₁₀alkylene-Y-C₆₋₁₀heteroaryl wherein -Y- is -CH₂-, -NH-, -O- or - S-;
symbol ^{∗} represents the bonding position;
m is an integer from 0 to 4;
-T- is absent, C₁₋₃alkylene or C₂₋₃alkenylene;
-T'- is C₁₋₃alkylene; and
wherein the heteroaryl contains at least one heteroatom, each heteroatom being independently S, N or O;
wherein the alkyl, alkenyl, alkoxy, cycloalkyl, aryl and heteroaryl are each independently unsubstituted or substituted with at least one substituent;
wherein the substituent is each independently a halogen, amino, nitro, nitroso, linear or branched C₁₋₁₅ alkyl, linear or branched C₁₋₁₅ alkoxy or C₃₋₁₀cycloalkyl; and when R_{b} is H, the tautomers are included,
or a pharmaceutically acceptable salt thereof.

In one embodiment, the compound is a compound of formula (I),
wherein n is 0 or 1;
X is -S-, -S(=O)- or -NRₙ-; wherein
Rₙ is H or 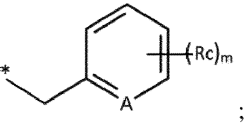
A is -CH, -CR_{c} or N;
Rₐ is -ORₐ₂, -O-C(=O)Rₐ₃ or -O-C(=O)-T-ORₐ₄; wherein
Rₐ₂ is H, linear or branched C₁₋₁₅alkyl, phosphonate or diarylphosphonate;
Rₐ₃ and Rₐ₄ are independently linear or branched C₁₋₁₅alkyl, linear or branched C₂₋₁₅alkenyl, - T-C₃₋₁₀cycloalkyl, -T-NHRₐ₃ₚ, -T-C₃₋₁₀cycloalkenyl, -T-C₆₋₁₀aryl, -T-C₅₋₁₀heteroaryl, -T-NH-C(=O)-O-C₁₋₁₀alkyl, -T-adamantyl or -C₁₋₃alkylene-C₆₋₁₀aryl where the alkylene is substituted with -T-NHRₐ₃ₚ;
Rₐ₃ₚ is H, Fmoc or Boc;
R_{b} is H, linear or branched C₁₋₁₅alkyl, linear or branched C₂₋₁₅alkenyl, C₁₋₃alkoxy-C₁₋₁₅alkyl-, -T'-C₃₋₁₀cycloalkyl, -T'-C₃₋₁₀cycloalkenyl, -T'-C₆₋₁₀ aryl or -T'-C₅₋₁₀heteroaryl;
R_{c} each is independently halogen, linear or branched C₁₋₁₅alkyl, linear or branched C₁₋₁₅alkoxyl, hydroxyl group, or -C₁₋₁₀alkylene-Y-C₆₋₁₀heteroaryl wherein -Y- is -CH₂-, -NH-, - O- or -S-;
symbol ^{∗} represents the bonding position;
m is an integer from 0 to 4;
-T- is absent, C₁₋₃alkylene or C₂₋₃alkenylene;
-T'- is C₁₋₃alkylene or C₂₋₃alkenylene; and
wherein the heteroaryl contains at least one heteroatom, each heteroatom being independently S, N or O;
wherein the alkyl, alkenyl, alkoxy, cycloalkyl, aryl, heteroaryl, alkylene and alkenylene are each independently unsubstituted or substituted with at least one substituent;
wherein the substituent is each independently halogen, amino, nitro, nitroso, linear or branched C₁₋₁₅ alkyl, or linear or branched C₁₋₁₅ alkoxy or C₃₋₁₀cycloalkyl; and when R_{b} is H, the tautomers are included,
or a pharmaceutically acceptable salt thereof.

In a further embodiment, the compound is a compound of formula (I), wherein
n is 0;
X is -S(=O)-;
AisN;
Rₐ is -ORₐ₂, -O-C(=O)Rₐ₃ or -O-C(=O)-T-ORₐ₄, wherein Rₐ₂ is H, linear or branched C₁₋₁₅alkyl, phosphonate or diarylphosphonate;
Rₐ₃ and Rₐ₄ are independently linear or branched C₁₋₁₅alkyl, linear or branched C₂₋₁₅alkenyl, - T-C₃₋₁₀cycloalkyl, -T-NHRₐ₃ₚ, -T-C₃₋₁₀cycloalkenyl, -T-C₆₋₁₀aryl, -T-C₅₋₁₀heteroaryl, -T-NH-C(=O)-O-C₁-₁₀alkyl, -T-adamantyl or -C₁₋₃alkylene-C₆₋₁₀aryl where the alkylene is substituted with -T-NHRₐ₃ₚ; Rₐ₃ₚ is H, Fmoc or Boc;
R_{b} is H;
m is 3; and
R_{c} each is independently linear or branched C₁₋₁₅alkyl, linear or branched C₁₋₁₅alkoxyl;
or a pharmaceutically acceptable salt thereof.

In one embodiment, the compound is a compound of formula (I), wherein n is 0.

In one embodiment, the compound is a compound of formula (I), wherein m is an integer from 0 to 3.

In one embodiment, the compound is a compound of formula (I), wherein
n is 0;
X is -S(=O);
A is N;
Rₐ is -O-C(=0)Rₐ₃ or -O-C(=O)-T-ORₐ₄; wherein
   Rₐ₃ is a linear or branched C₁₋₁₅alkyl, linear or branched C₂₋₁₅alkenyl, -T-C₃₋₁₀cycloalkyl, -T-NHRₐ₃ₚ, -T-C₃₋₁₀cycloalkenyl, -T-C₆₋₁₀aryl, -T-C₅₋₁₀heteroaryl, -T-NH-C(=O)-O-C₁₋₁₀alkyl, -T-adamantyl or -C₁₋₃alkylene-C₆₋₁₀aryl where the alkylene is substituted with -T-NHRₐ₃ₚ;
   Rₐ₄ is a linear or branched C₁₋₁₅alkyl, linear or branched C₂₋₁₅alkenyl, -T-C₃₋₁₀cycloalkyl, -T-NHRₐ₃ₚ, -T-C₃₋₁₀cycloalkenyl, -T-C₆₋₁₀aryl, -T-C₅₋₁₀heteroaryl, -T-NH-C(=O)-O-C₁₋₁₀alkyl, -T-adamantyl or -C₁₋₃alkylene-C₆₋₁₀aryl where the alkylene is substituted with -T-NHRₐ₃ₚ;
   Rₐ₃ₚ is H or an *N*-protecting group selected from Fmoc or Boc;
or a pharmaceutically acceptable salt thereof.

In one embodiment, the compound is a compound of formula (I), wherein
A is N;
R_{b} is H;
m is 3; and
R_{c} each is independently linear or branched C₁₋₁₅alkyl, linear or branched C₁₋₁₅alkoxyl; or a pharmaceutically acceptable salt.

In some embodiments, the compound is a compound of formula (I), wherein Rₐ is -O-C(=O)Rₐ₃ wherein Rₐ₃ is adamantyl; linear or branched C₁₋₁₀alkyl unsubstituted or substituted by halogen; C₁₋₄alkoxy; -C₆₋₁₀aryl unsubstituted or substituted by C₁₋₁₀ alkyl, nitro, C₁₋₁₅alkoxy or halogen; C₃₋₁₀cycloalkyl; -C₃₋₁₀cycloalkenyl; linear or branched C₂₋₁₀alkenyl; -C₅₋₁₀heteroaryl; -C₁₋₃alkylene-C₃₋₁₀cycloalkyl; C₂₋₃alkenylene-C₆₋₁₀aryl wherein C₆₋₁₀aryl is unsubstituted or substituted by halogen; -O-C(=O)-O-C₁₋₁₀alkyl.

In some embodiments, the compound is a compound of formula (I), wherein Rₐ is - O-C(=O)Rₐ₃ wherein Rₐ₃ is adamantyl; linear or branched C₁₋₈alkyl unsubstituted or substituted by halogen; C₁₋₄alkoxy; -phenyl unsubstituted or substituted by C₁₋₆ alkyl, nitro, C₁₋₄alkoxy or halogen; C₃₋₆cycloalkyl; -C₃₋₆cycloalkenyl; linear or branched C₂₋₆alkenyl; -C₅₋₆heteroaryl; -C₁₋₃alkylene-C₃₋₆cycloalkyl; C₂₋₃alkenylene-phenyl wherein phenyl is unsubstituted or substituted by halogen; -O-C(=O)-O-C₁₋₄alkyl.

In some embodiments, the compound is a compound of formula (I), wherein Rₐ is -O-C(=O)-C₁₋₆alkyl, -O-C(=O)-C₁₋₃alkylene-NHFmoc,-O-C(=O)-C₁₋₃alkylene-NHBoc or -O-C(=O)-NH-C(=O)-O-C₁₋₁₀alkyl.

In some embodiments, the compound is a compound of formula (I), wherein R_{c} each is independently halogen, linear or branched C₁₋₆alkyl, linear or branched C₁₋₆alkoxyl, or -C₁₋₁₀alkenylene-Y-C₆₋₁₀heteroaryl; wherein Y is S and C₆₋₁₀heteroaryl is unsubstituted or substituted by C₁₋₁₅alkyl, C₁₋₁₅alkoxy, -OH, -NH₂, -NO₂ or halogen.

In some embodiments, the compound is a compound of formula (I), wherein:
X is -S-;
A is N;
Rₐ is -C(=O)ORₐ₁; wherein Rₐ₁ is H or linear or branched C₁₋₁₅alkyl;
or a pharmaceutically acceptable salt thereof.

In some embodiments, the compound is a compound of formula (I), wherein Rₐ is - C(=O)OH, or -C(=O)OC₁₋₄alkyl.

In some embodiments, the compound is a compound of formula (I), wherein R_{c} each is independently halogen, linear or branched C₁₋₆alkyl, linear or branched C₁₋₆alkoxyl, or -C₁₋₁₀alkenylene-Y-C₆₋₁₀heteroaryl; wherein Y is S and C₆₋₁₀heteroaryl is unsubstituted or substituted by C₁₋₁₅alkyl, C₂₋₁₅alkenyl, C₁₋₁₅alkoxy, -OH, -NH₂, -NO₂ or halogen.

In some embodiments, the compound is a compound of formula (I), wherein Rₐ is - C(=O)OH, -C(=O)OC₁₋₄alkyl, H, -ORₐ₂ wherein Rₐ₂ is H, linear or branched C₁₋₁₀alkyl; -O-C(=O)Rₐ₃ wherein Rₐ₃ is independently tert-butyl; linear or branched C₁₋₁₀alkyl unsubstituted or substituted by halogen, tert-butyl or amino; linear or branched C₂₋₁₀alkenyl; C₁₋₄alkoxy; C₃₋₁₀cycloalkyl; -C₁₋₃alkylene-C₃₋₁₀cycloalkyl; -C₃₋₁₀cycloalkenyl; -C₆₋₁₀aryl unsubstituted or substituted by C₁₋₁₀ alkyl, nitro, C₁₋₁₅alkoxy or halogen; -C₅₋₁₀heteroaryl unsubstituted or substituted by C₁₋₁₀alkoxy; C₂₋₃alkenylene-C₆₋₁₀aryl wherein C₆₋₁₀aryl is unsubstituted or substituted by halogen; -C₁₋₃alkylene-NH--C(=O)-O-C₁₋₁₀alkyl; or adamantyl; or -O-C(=O)-O-C₁₋₁₀alkyl.

In some embodiments, the compound is a compound of formula (I), wherein Rₐ is -O-C₁₋₁₀alkyl or -O-C(=O)Rₐ₃ wherein Rₐ₃ is a tert-butyl; adamantyl; linear or branched C₁₋₁₀alkyl unsubstituted or substituted by halogen or tert-butyl; C₁₋₄alkoxy; -C₆₋₁₀aryl unsubstituted or substituted by C₁₋₁₀ alkyl, nitro, C₁₋₁₅alkoxy or halogen; C₃₋₁₀cycloalkyl; -C₃₋₁₀cycloalkenyl; linear or branched C₂₋₁₀alkenyl; -C₅₋₁₀heteroaryl; -C₁₋₃alkylene-C₃₋₁₀cycloalkyl; C₂₋₃alkenylene-C₆₋₁₀aryl wherein C₆₋₁₀aryl is unsubstituted or substituted by halogen; -O-C(=O)-O-C₁₋₁₀alkyl. In some embodiments, Rₐ is -O-C₁₋₄alkyl, -O-tert-butyloxycarbonyl or -O-C(=O)Rₐ₃ wherein Rₐ₃ is tert-butyl; adamantyl; linear or branched C₁₋₈alkyl unsubstituted or substituted by halogen or tert-butyl; C₁₋₄alkoxy; -phenyl unsubstituted or substituted by C₁₋₆ alkyl, nitro, C₁₋₄alkoxy or halogen; C₃₋₆cycloalkyl; -C₃₋₆cycloalkenyl; linear or branched C₂₋₆alkenyl; -C₅₋₆heteroaryl; -C₁₋₃alkylene-C₃₋₆cycloalkyl; C₂₋₃alkenylene-phenyl wherein phenyl is unsubstituted or substituted by halogen; - O-C(=O)-O-C₁₋₄alkyl. In some further embodiments, C₃₋₆cycloalkyl is cyclopropyl or cyclohexyl. In some further embodiments, -C₃₋₁₀cycloalkenyl is cyclohexenyl.

In some further embodiments, heteroaryl is pyrrolidinyl, pyrrolinyl, pyrazolidinyl, imidazolidinyl, pyrazolinyl, imidazolinyl, pyrazolyl, imidazolyl, tetrahydrofuranyl, furanyl, dioxolanyl, tetrahydrothiophenyl, thiophenyl, oxazolyl, isoxazolyl, isothiazolyl, thiazolyl, oxathiolanyl, piperidinyl, pyridinyl, piperazinyl, pyridazinyl, pyrimidinyl, pyrazinyl, tetrahydropyranyl, pyranyl, dioxanyl, thianyl, thiopyranyl, morpholinyl, oxazinyl or thiazinyl. In further embodiments, heteroaryl is furanyl, isoxazolyl or thiophenyl.

In some embodiments, the compound is a compound of formula (I), wherein Rₐ is -OH, -COOH, -O-phosphate, -O-C₁₋₆alkyl or -O-C(=O)-C₁₋₆alkyl, -O-C(=O)-C₁₋₄alkylene-NH(Fmoc or Boc), or -O-C(=O)-NH-C(=O)-O-C₁₋₁₀alkyl.

In some embodiments, the compound is a compound of formula (I), wherein R_{c} each is independently linear or branched C₁₋₆alkyl or linear or branched C₁₋₆alkoxyl. In some embodiments, R_{c} each is independently halogen, linear or branched C₁₋₆alkyl, linear or branched C₁₋₆alkoxyl, or -C₁₋₁₀alkenylene-Y-C₆₋₁₀heteroaryl; wherein Y is S and C₆₋₁₀heteroaryl is unsubstituted or substituted by C₁₋₁₅alkyl (preferably C₁₋₄alkyl), C₁₋₁₅alkenyl (preferably C₂₋₄alkyl), C₁₋₁₅alkoxy (preferably C₁₋₄alkoxy), -OH, -NH₂, -NO₂ or halogen. In a further embodiment, -C₁₋₁₀alkenylene-Y-C₆₋₁₀heteroaryl is

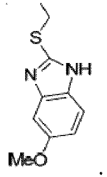

In some embodiments of formula (I), the compounds include but are not limited to:

| **NCTU-SUN-ID** | **Chemical Structure** |
|---|---|
| RS-D7 | 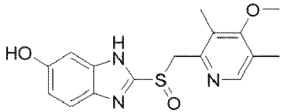 |
| | 5 -O-Desmethyl-Omeprazole |
| Esomeprazole | 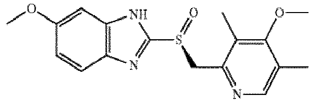 |
| | (S)-(-)-5-Methoxy-2-[(4-methoxy-3,5-dimethylpyridin-2-yl)methylsulfinyl]-3H-benzoimidazole |
| 13001 | 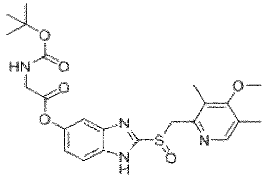 |
| | 2-(((4-methoxy-3,5-dimethylpyridin-2-yl)methyl)sulfinyl)-1H-benzo[d]imidazol-6-yl 2-((tert-butoxycarbonyl)amino)acetate |
| 26090 | 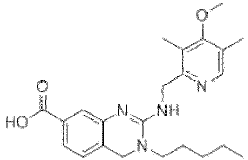 |
| | 2-(((4-methoxy-3,5-dimethylpyridin-2-yl)methyl)amino)-3-pentyl-3,4-dihydroquinazoline-7-carboxylic acid |
| 21115 | 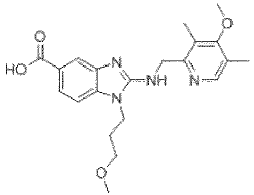 |
| | 2-(((4-methoxy-3,5-dimethylpyridin-2-yl)methyl)amino)-1-(3-methoxypropyl)-1H-benzo[d]imidazole-5-carboxylic acid |
| 25030 | 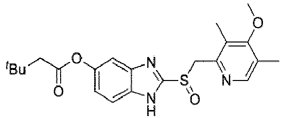 |
| | (2-(((4-methoxy-3,5-dimethylpyridin-2-yl)methyl)sulfinyl)-1H-benzo[d]imidazol-5-yl 3,3-dimethylbutanoate) |
| 28096 | 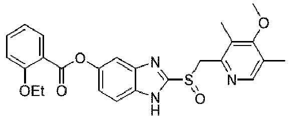 |
| | (2-(((5-methoxy-4,6-dimethylpyridin-2-yl)methyl)sulfinyl)-1H-benzo[d]imidazol-5-yl 2-ethoxybenzoate) |
| 12093 | 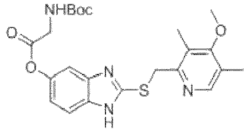 |
| | 2-(((4-methoxy-3,5-dimethylpyridin-2-yl)methyl)thio)-1H-benzo[d]imidazol-5-yl (tert-butoxycarbonyl)glycinate |
| 25016 | 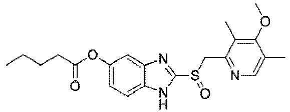 |
| | (2-(((4-methoxy-3,5-dimethylpyridin- 2-yl)methyl)sulfinyl)-1H-benzo[d]imidazol-5-yl pentanoate) |
| 12130 | 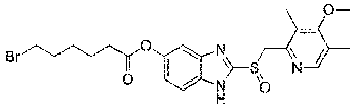 |
| | (2-(((4-methoxy-3,5-dimethylpyridin-2-yl)methyl)sulfinyl)-1H-benzo[d]imidazol-5-yl 6-bromohexanoate) |
| 27077 | 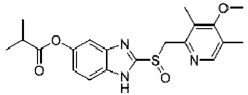 |
| | 2-(((4-methoxy-3,5-dimethylpyridin-2-yl)methyl)sulfinyl)-1H-benzo[d]imidazol-5-yl isobutyrate |
| 27079 | 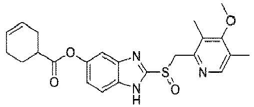 |
| | 2-(((4-methoxy-3,5-dimethylpyridin-2-yl)methyl)sulfinyl)-1H-benzo[d]imidazol-5-yl cyclohex-3-enecarboxylate |
| 26089 | 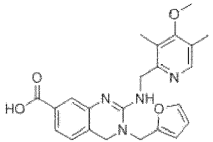 |
| | 3-(furan-2-ylmethyl)-2-(((4-methoxy-3,5-dimethylpyridin-2-yl)methyl)amino)-3,4-dihydroquinazoline-7-carboxylic acid |
| 21129 | 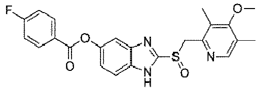 |
| | (2-(((4-methoxy-3,5-dimethylpyridin-2-yl)methyl)sulfinyl)-1H-benzo[d]imidazol-5-yl 4-fluorobenzoate) |
| 25032 | 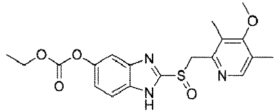 |
| | (ethyl (2-(((4-methoxy-3,5-dimethylpyridin-2-yl)methyl)sulfinyl)-1H-benzo[d]imidazol-5-yl) carbonate) |
| 26098 | 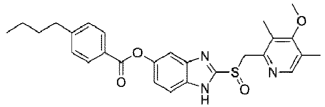 |
| | (2-(((4-methoxy-3,5-dimethylpyridin-2-yl)methyl)sulfinyl)-1H-benzo[d]imidazol-5-yl 4-butylbenzoate) |
| 21127 | 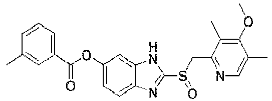 |
| | (2-(((4-methoxy-3,5-dimethylpyridin-2-yl)methyl)sulfinyl)-1H-benzo[d]imidazol-5-yl 3-methylbenzoate) |
| 25017 | 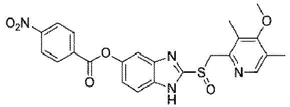 |
| | (2-(((4-methoxy-3,5-dimethylpyridin-2-yl)methyl)sulfinyl)-1H-benzo[d]imidazol-5-yl 4-nitrobenzoate) |
| 12128 | 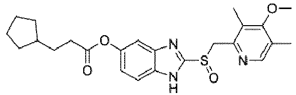 |
| | (2-(((4-methoxy-3,5-dimethylpyridin-2-yl)methyl)sulfinyl)-1H-benzo[d]imidazol-5-yl 3-cyclopentylpropanoate) |
| 26071 | 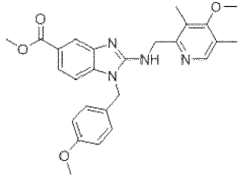 |
| | Methyl 2-(((4-methoxy-3,5-dimethylpyridin-2-yl)methyl)amino)-1-(4-methoxybenzyl)-1H-benzo[d]imidazole-5-carboxylate |
| 11021 | 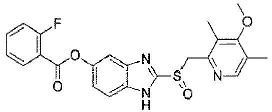 |
| | (2-(((4-methoxy-3,5-dimethylpyridin-2-yl)methyl)sulfinyl)-1H-benzo[d]imidazol-5-yl 2-fluorobenzoate) |
| 21118 | 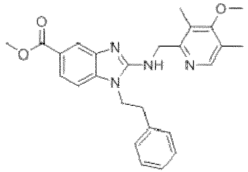 |
| | Methyl 2-(((4-methoxy-3,5-dimethylpyridin-2-yl)methyl)amino)-1- phenethyl-1H-benzo[d]imidazole-5-carboxylate |
| 26070 | 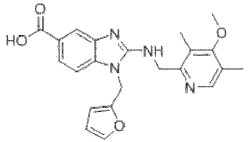 |
| | 1-(furan-2-ylmethyl)-2-(((4-methoxy-3,5-dimethylpyridin-2-yl)methyl)amino)-1H-benzo[d]imidazole-5-carboxylic acid |
| 25029 | 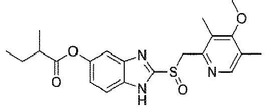 |
| | (2-(((4-methoxy-3,5-dimethylpyridin-2-yl)methyl)sulfinyl)-1H-benzo[d]imidazol-5-yl 2-methylbutanoate) |
| 12129 | 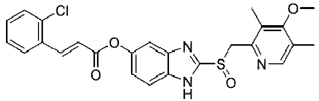 |
| | (2-(((4-methoxy-3,5-dimethylpyridin-2-yl)methyl)sulfinyl)-1H-benzo[d]imidazol-5-yl (E)-3-(2-chlorophenyl)acrylate) |
| 11023 | 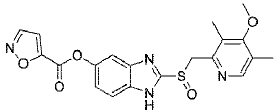 |
| | (2-(((4-methoxy-3,5-dimethylpyridin-2-yl)methyl)sulfinyl)-1H-benzo[d]imidazol-5-yl isoxazole-5-carboxylate) |
| 26096 | 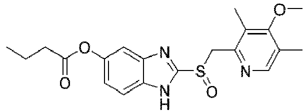 |
| | (2-(((4-methoxy-3,5-dimethylpyridin-2-yl)methyl)sulfinyl)-1H-benzo[d]imidazol-5-yl butyrate) |
| 25027 | 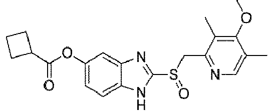 |
| | (2-(((4-methoxy-3,5-dimethylpyridin-2-yl)methyl)sulfinyl)-1H-benzo[d]imidazol-5-yl cyclobutanecarboxylate) |
| 28092 | 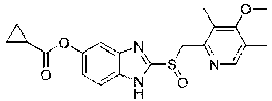 |
| | (2-(((4-methoxy-3,5-dimethylpyridin-2-yl)methyl)sulfinyl)-1H-benzo[d]imidazol-5-yl cyclopropanecarboxylate) |
| 12088 | 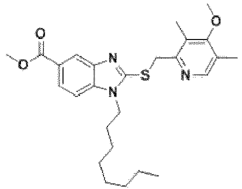 |
| | methyl 2-(((4-methoxy-3,5-dimethylpyridin-2-yl)methyl)thio)-1-octyl-1H-benzo[d]imidazole-5-carboxylate |
| 12127 | 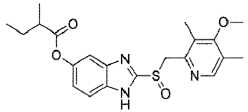 |
| | (2-(((4-methoxy-3,5-dimethylpyridin-2-yl)methyl)sulfinyl)-1H-benzo[d]imidazol-5-yl 2-methylbutanoate) |
| 21117 | 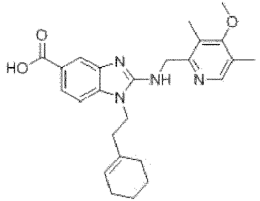 |
| | 1-(2-(cyclohex-1-en-1-yl)ethyl)-2-(((4-methoxy-3,5-dimethylpyridin-2- yl)methyl)amino)-1H-benzo[d]imidazole-5-carboxylic acid |
| 12084 | 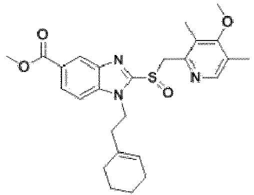 |
| | Methyl 1-(2-(cyclohex-1-en-1-yl)ethyl)-2-(((4-methoxy-3,5-dimethylpyridin-2-yl)methyl)sulfinyl)-1H-benzo[d]imidazole-5-carboxylate |
| 21126 | 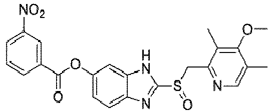 |
| | (2-(((4-methoxy-3,5-dimethylpyridin-2-yl)methyl)sulfinyl)-1H-benzo[d]imidazol-5-yl 3-nitrobenzoate) |
| 26097 | 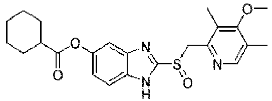 |
| | (2-(((4-methoxy-3,5-dimethylpyridin-2-yl)methyl)sulfinyl)-1H-benzo[d]imidazol-5-yl cyclohexanecarboxylate) |
| 21110 | 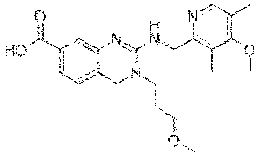 |
| | 2-(((4-methoxy-3,5-dimethylpyridin-2-yl)methyl)amino)-3- (3-methoxypropyl)-3,4-dihydroquinazoline-7-carboxylic acid |
| 21116 | 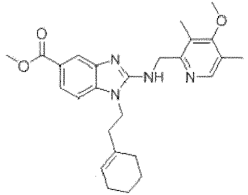 |
| | Methyl 1-(2-(cyclohex-1-en-1-yl)ethyl)-2-(((4-methoxy-3,5- dimethylpyridin-2-yl)methyl)amino)- 1H-benzo[d]imidazole-5-carboxylate |
| 21120 | 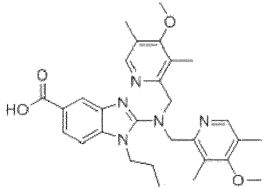 |
| | 2-(bis((4-methoxy-3,5-dimethylpyridin-2-yl)methyl) amino)-1-propyl- 1H-benzo[d]imidazole-5-carboxylic acid |
| 21121 | 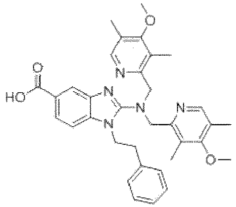 |
| | 2-(bis((4-methoxy-3,5-dimethylpyridin-2-yl)methyl)amino)-1-phenethyl- 1H-benzo[d]imidazole-5-carboxylic acid |
| 22138 | 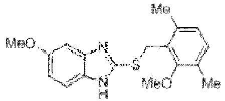 |
| | 5-methoxy-2-((2-methoxy-3,6-dimethylbenzyl)thio)-1H-benzo[d]imidazole |
| 25015 | 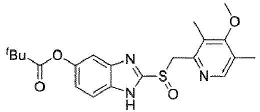 |
| | (2-(((4-methoxy-3,5-dimethylpyridin-2-yl)methyl)sulfinyl)-1H-benzo[d]imidazol-5-yl pivalate) |
| 28094 | 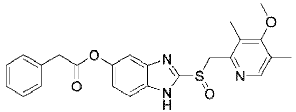 |
| | (2-(((4-methoxy-3,5-dimethylpyridin-2-yl)methyl)sulfinyl)-1H-benzo[d]imidazol-5-yl 2-phenylacetate) |
| 28091 | 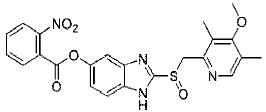 |
| | (2-(((4-methoxy-3,5-dimethylpyridin-2-yl)methyl)sulfinyl)-1H-benzo[d]imidazol-5-yl 2-nitrobenzoate) |
| 21130 | 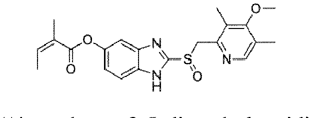 |
| | (2-(((4-methoxy-3,5-dimethylpyridin-2-yl)methyl)sulfinyl)-1H-benzo[d]imidazol-5-yl (Z)-2-methylbut-2-enoate) |
| 21103 | 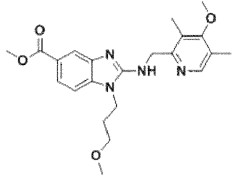 |
| | Methyl 2-(((4-methoxy-3,5-dimethylpyridin-2-yl)methyl)amino)-1-(3-methoxypropyl)-1H-benzo[d]imidazole-5-carboxylate |
| 21122 | 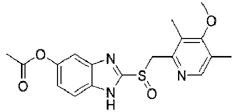 |
| | (2-(((4-methoxy-3,5-dimethylpyridin-2-yl)methyl)sulfinyl)-1H-benzo[d]imidazol-5-yl acetate) |
| 11022 | 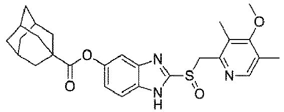 |
| | (2-(((4-methoxy-3,5-dimethylpyridin-2-yl)methyl)sulfinyl)-1H-benzo[d]imidazol-5-yl (3r,5r,7r)-adamantane-1-carboxylate) |
| 11030 | 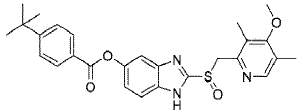 |
| | (2-(((4-methoxy-3,5-dimethylpyridin-2-yl)methyl)sulfinyl)-1H-benzo[d]imidazol-5-yl 4-(tert-butyl)benzoate) |
| 21102 | 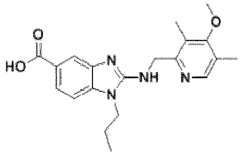 |
| | 2-(((4-methoxy-3,5-dimethylpyridin-2-yl)methyl)amino)-1-propyl- 1H-benzo[d]imidazole-5-carboxylic acid |
| 21132 | 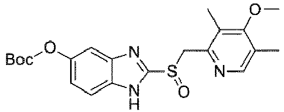 |
| | tert-butyl (2-(((4-methoxy-3,5-dimethylpyridin-2-yl)methyl)sulfinyl)-1H-benzo [d]imidazol-5 -yl) carbonate |
| 12123 | 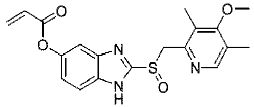 |
| | (2-(((4-methoxy-3,5-dimethylpyridin-2-yl)methyl)sulfinyl)-1H-benzo[d]imidazol-5-yl acrylate) |
| 13084 | 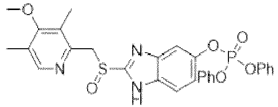 |
| | 2-(((4-methoxy-3,5-dimethylpyridin-2-yl)methyl)sulfinyl)-1H-benzo[d]imidazol-5-yl diphenyl phosphate |
| 12094 | 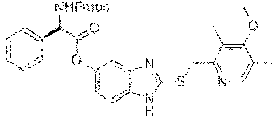 |
| | 2-(((4-methoxy-3,5-dimethylpyridin-2-yl)methyl)thio)-1H benzo [d] -imidazole-5 -yl (S)-2-((((9H-fluoren-9-yl)methoxy)carbonyl)amino)-2-phenylacetate |
| 21105 | 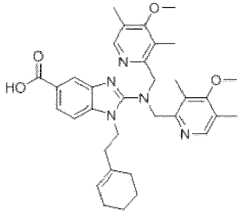 |
| | 2-(bis((4-methoxy-3,5-dimethylpyridin-2-yl)methyl)amino)-1-(2-(cyclohex-1-en-1-yl)ethyl)-1H-benzo[d]imidazole-5-carboxylic acid |
| 12124 | 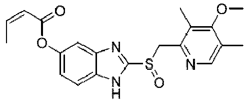 |
| | (2-(((4-methoxy-3,5-dimethylpyridin-2-yl)methyl)sulfinyl)-1H-benzo[d]imidazol-5-yl (Z)-but-2-enoate) |
| 12122 | 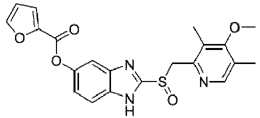 |
| | (2-(((4-methoxy-3,5-dimethylpyridin-2-yl)methyl)sulfinyl)-1H-benzo[d]imidazol-5-yl furan-2-carboxylate) |
| 26072 | 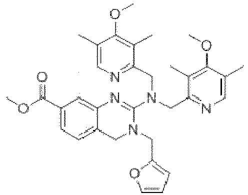 |
| | Methyl 2-(bis((4-methoxy-3,5-dimethylpyridin-2-yl)methyl)amino)-3-(furan-2-ylmethyl)-3,4-dihydroquinazoline-7-carboxylate |
| 22140 | 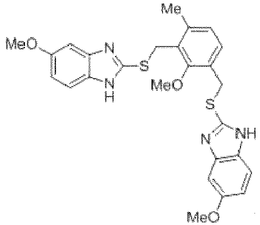 |
| | 2,2'-(((2-methoxy-4-methyl-1,3-phenylene)bis(methylene))bis(sulfanediyl))bis(5-methoxy-1H-benzo[d]imidazole) |
| 21133 | 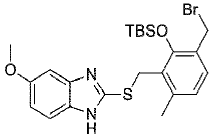 |
| | 2-((3-(bromomethyl)-2-((tert-butyldimethylsilyl)oxy)-6-methylbenzyl)thio)-5-methoxy-1H-benzo[d]imidazole |
| 21125 | 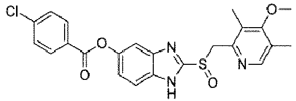 |
| | 2-(((4-methoxy-3,5-dimethylpyridin-2-yl)methyl)sulfinyl)-1H-benzo[d]imidazol-5-yl 4-chlorobenzoate |
| 27078 | 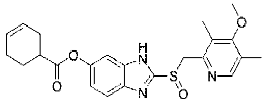 |
| | (2-(((4-methoxy-3,5-dimethylpyridin-2-yl)methyl)sulfinyl)-1H-benzo[d]imidazol-5-yl cyclohex-3-ene-1-carboxylate) |
| 11020 | 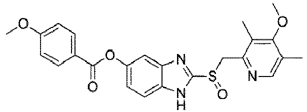 |
| | (2-(((4-methoxy-3,5-dimethylpyridin-2-yl)methyl)sulfinyl)-1H-benzo[d]imidazol-5-yl 4-methoxybenzoate) |
| 26076 | 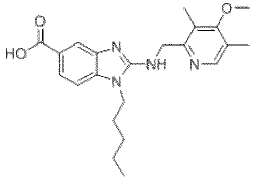 |
| | 2-(((4-methoxy-3,5-dimethylpyridin-2-yl)methyl)amino)-1-pentyl-1H-benzo [d]imidazole-5 -carboxylic acid |
| 25031 | 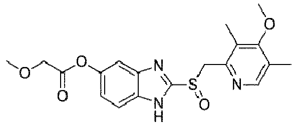 |
| | (2-(((4-methoxy-3,5-dimethylpyridin-2-yl)methyl)sulfinyl)-1H-benzo[d]imidazol-5-yl 2-methoxyacetate) |
| 21128 | 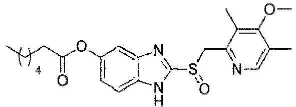 |
| | 2-(((4-methoxy-3,5-dimethylpyridin-2-yl)methyl)sulfinyl)-1H-benzo[d]imidazol-5-yl heptanoate |
| 27076 | 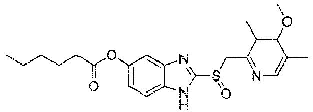 |
| | 2-(((4-methoxy-3,5-dimethylpyridin-2-yl)methyl)sulfinyl)-1H-benzo[d]imidazol-5-yl hexanoate |
| 12083 | 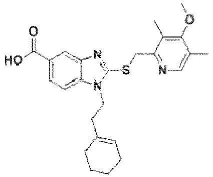 |
| | 1-(2-(cyclohex-1-en-1-yl)ethyl)-2-(((4-methoxy3,5-dimethylpyridin-2-yl)methyl)thio)-1H-benzo[d]imidazole-5-carboxylic acid |
| 21119 | 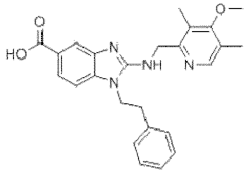 |
| | 2-(((4-methoxy-3,5-dimethylpyridin-2-yl)methyl)amino)-1-phenethyl- 1H-benzo[d]imidazole-5-carboxylic acid |
| 21104 | 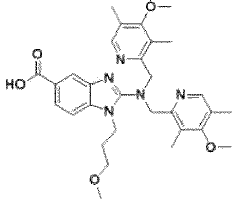 |
| | 2-(bis((4-methoxy-3,5-dimethylpyridin-2-yl)methyl)amino)-1- (3-methoxypropyl)-1H-benzo[d]imidazole-5-carboxylic acid |
| 21106 | 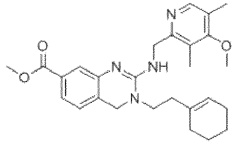 |
| | Methyl 3-(2-(cyclohex-1-en-1-yl)ethyl)-2-(((4-methoxy-3,5- dimethylpyridin-2-yl)methyl)amino)-3,4-dihydroquinazoline-7-carboxylate |
| 26077 | 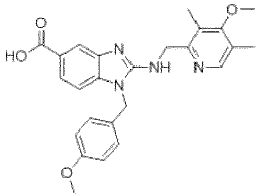 |
| | 2-(((4-methoxy-3,5-dimethylpyridin-2-yl)methyl)amino)-1-(4-methoxybenzyl)-1H-benzo[d]imidazole-5-carboxylic acid |
| 26066 | 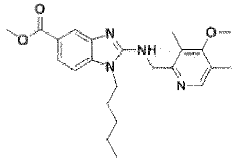 |
| | Methyl 2-(((4-methoxy-3,5-dimethylpyridin-2-yl)methyl)amino)-1-pentyl-1H-benzo [d]imidazole-5-carboxylate |
| 12125 | 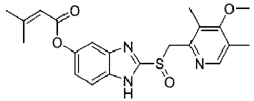 |
| | (2-(((4-methoxy-3,5-dimethylpyridin-2-yl)methyl)sulfinyl)-1H-benzo[d]imidazol-5-yl 3-methylbut-2-enoate) |
| 26065 | 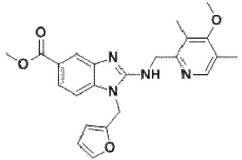 |
| | Methyl 1-(furan-2-ylmethyl)-2-(((4-methoxy-3,5-dimethylpyridin-2-yl)methyl)amino)-1H-benzo[d]imidazole-5-carboxylate |
| 26079 | 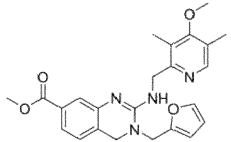 |
| | Methyl 3-(furan-2-ylmethyl)-2-(((4-methoxy-3,5-dimethylpyridin-2-yl)methyl)amino)-3,4-dihydroquinazoline-7-carboxylate |
| 25028 | 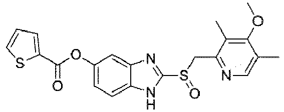 |
| | (2-(((4-methoxy-3,5-dimethylpyridin-2-yl)methyl)sulfinyl)-1H-benzo[d]imidazol-5-yl thiophene-2-carboxylate) |
| 26092 | 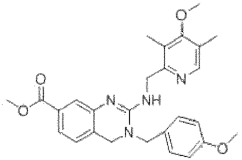 |
| | Methyl 2-(((4-methoxy-3,5-dimethylpyridin-2-yl)methyl)amino)-3-(4-methoxybenzyl)- 3,4-dihydroquinazoline-7-carboxylate |
| 21124 | 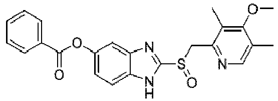 |
| | (2-(((4-methoxy-3,5-dimethylpyridin-2-yl)methyl)sulfinyl)-1H-benzo[d]imidazol-5-yl benzoate) |
| 28093 | 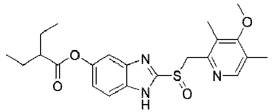 |
| | (2-(((4-methoxy-3,5-dimethylpyridin-2-yl)methyl)sulfinyl)-1H-benzo[d]imidazol-5-yl 2-ethylbutanoate) |
| 26091 | 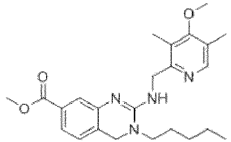 |
| | Methyl 2-(((4-methoxy-3,5-dimethylpyridin-2-yl)methyl)amino)-3-pentyl-3,4-dihydroquinazoline-7-carboxylate |
| 22139 | 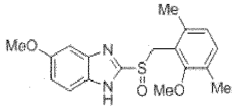 |
| | 5-methoxy-2-((2-methoxy-3,6-dimethylbenzyl)sulfinyl)-1H-benzo[d]imidazole |
| 22141 | 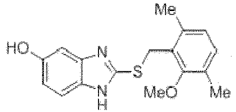 |
| | 2-((2-methoxy-3,6-dimethylbenzyl)thio)-1H-benzo[d]imidazol-5-ol |
| 28087 | 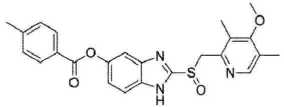 |
| | (2-(((4-methoxy-3,5-dimethylpyridin-2-yl)methyl)sulfinyl)-1H-benzo[d]imidazol-5-yl 4-methylbenzoate) |
| 21123 | 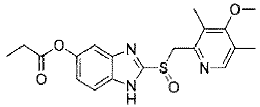 |
| | (2-(((4-methoxy-3,5-dimethylpyridin-2-yl)methyl)sulfinyl)-1H-benzo[d]imidazol-5-yl propionate) |
| 12092 | 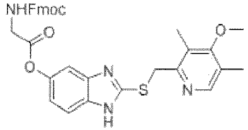 |
| | 2-(((4-methoxy-3,5-dimethylpyridin-2-yl)methyl)thio)-1H-benzo[d]imidazol-5-yl (((9H-fluoren-9-yl)methoxy)carbonyl)glycinate |
| 21098 | 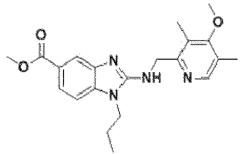 |
| | Methyl 2-(((4-methoxy-3,5-dimethylpyridin-2-yl)methyl) amino)- 1-propyl-1H-benzo[d]imidazole-5-carboxylate |
| 21131 | 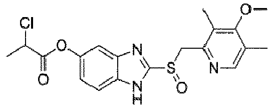 |
| | (2-(((4-methoxy-3,5-dimethylpyridin-2-yl)methyl)sulfinyl)-1H-benzo[d]imidazol-5-yl 2-chloropropanoate) |
| 12082 | 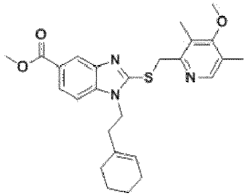 |
| | Methyl 1-(2-(cyclohex-1-en-1-yl)ethyl)-2-(((4-methoxy-3,5-dimethylpyridin-2-yl)methyl)thio)-1H-benzo[d]imidazole-5-carboxylate |
| 28095 | 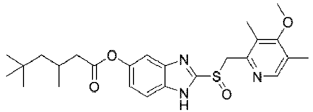 |
| | (2-(((4-methoxy-3,5-dimethylpyridin-2-yl)methyl)sulfinyl)-1H-benzo[d]imidazol-5-yl 3,5,5-trimethylhexanoate |
| 11031 | 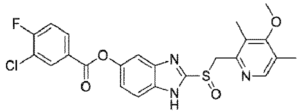 |
| | (2-(((4-methoxy-3,5-dimethylpyridin-2-yl)methyl)sulfinyl)-1H-benzo[d]imidazol-5-yl 3-chloro-4-fluorobenzoate) |

or a pharmaceutically acceptable salt thereof.

The present disclosure encompasses all stereoisomeric forms of the compounds of Formula I, Formula I-a and Formula I-b. Centers of asymmetry that are present in the compounds of Formula I, Formula I-a and Formula I-b can all independently of one another have (R) configuration or (S) configuration. When bonds to the chiral carbon are depicted as straight lines in the structural Formulas of the disclosure, it is understood that both the (R) and (S) configurations of the chiral carbon, and hence both enantiomers and mixtures thereof, are embraced within the Formula. When a particular configuration is depicted, that entantiomer (either (R) or (S), at that center) is intended. Similarly, when a compound name is recited without a chiral designation for a chiral carbon, it is understood that both the (R) and (S) configurations of the chiral carbon, and hence individual enantiomers and mixtures thereof, are embraced by the name.

The present disclosure includes all possible enantiomers and diastereomers and mixtures of two or more stereoisomers, for example mixtures of enantiomers and/or diastereomers, in all ratios. Thus, enantiomers are a subject of the present disclosure in enantiomerically pure form, both as levorotatory and as dextrorotatory antipodes, in the form of racemates and in the form of mixtures of the two enantiomers in all ratios. In the case of a cis/trans isomerism the present disclosure includes both the cis form and the trans form as well as mixtures of these forms in all ratios. The preparation of individual stereoisomers can be carried out, if desired, by separation of a mixture by customary methods, for example by chromatography or crystallization, by the use of stereochemically uniform starting materials for the synthesis or by stereoselective synthesis. Optionally a derivatization can be carried out before a separation of stereoisomers. The separation of a mixture of stereoisomers can be carried out at an intermediate step during the synthesis of a compound of Formula (I), (I-a) or (I-b) or it can be done on a final racemic product. Absolute stereochemistry may be determined by X-ray crystallography of crystalline products or crystalline intermediates which are derivatized, if necessary, with a reagent containing a stereogenic center of known configuration. Where compounds of the present disclosure are capable of tautomerization, all individual tautomers as well as mixtures thereof are included in the scope of the present disclosure. The present disclosure includes all such isomers, as well as salts, solvates (including hydrates) and solvated salts of such racemates, enantiomers, diastereomers and tautomers and mixtures thereof.

The compounds used in the method of the present disclosure can be prepared using methods known to those skilled in the art. For example, the general synthetic scheme for preparing the compound of formula (I) and preparation procedures of the aforesaid compounds were disclosed in WO2018053161A1, which is incorporated herein by reference.

### Utilities

The compounds of formula (I), (I-a) or (I-b) or a pharmaceutically acceptable salt thereof, may be useful in the treatment of a disease or disorder mediated by NLRP3, preferably selected from autoinflammatory fever syndromes (e.g. CAPS), sickle cell disease, Type I or Type II diabetes and related complications (e.g. nephropathy, retinopathy), hyperoxaluria, gout, pseudogout (chondrocalcinosis), chronic liver disease, NASH, neuroinflammation-related disorders (e.g. multiple sclerosis, brain infection, acute injury, neurodegenerative diseases, Alzheimer's disease), atherosclerosis and cardiovascular risk (e.g. cardiovascular risk reduction (CvRR), hypertension), hidradenitis suppurativa, wound healing and scar formation, and cancer (e.g. colon cancer, lung cancer, myeloproliferative neoplasms, leukemias, myelodysplastic syndromes (MDS), myelofibrosis). In a further embodiment, the therapy is selected from a disease, which may be treated by inhibition of NLRP3 inflammasome. In another embodiment, the disease is selected from the afore-mentioned list, suitably inflammasome-related diseases or disorders, immune diseases, inflammatory diseases, auto-immune diseases, or auto-inflammatory diseases, for example, autoinflammatory fever syndromes (e.g cryopyrin-associated periodic syndrome), sickle cell disease, systemic lupus erythematosus (SLE), liver related disease/disorders (e.g. chronic liver disease, viral hepatitis, non-alcoholic steatohepatitis (NASH), alcoholic steatohepatitis, and alcoholic liver disease), inflammatory arthritis related disorders (e.g. gout, pseudogout (chondrocalcinosis), osteoarthritis, rheumatoid arthritis, arthropathy e.g acute, chronic), kidney related diseases (e.g. hyperoxaluria, lupus nephritis, Type I or Type II diabetes and related complications (e.g. nephropathy, retinopathy) hypertensive nephropathy, hemodialysis related inflammation), neuroinflammation-related diseases (e.g. multiple sclerosis, brain infection, acute injury, neurodegenerative diseases, Alzheimer's disease), cardiovascular or metabolic diseases/disorders (e.g. cardiovascular risk reduction (CvRR), hypertension, atherosclerosis, type I and type II diabetes and related complications, peripheral artery disease (PAD), acute heart failure), inflammatory skin diseases (e.g. hidradenitis suppurativa, acne), wound healing and scar formation, asthma, sarcoidosis, age-related macular degeneration, and cancer related diseases or disorders (e.g. colon cancer, lung cancer, myeloproliferative neoplasms, leukemias, myelodysplastic syndromes (MDS), myelofibrosis). In particular, autoinflammatory fever syndromes (e.g. CAPS), sickle cell disease, Type I or Type II diabetes and related complications (e.g. nephropathy, retinopathy), hyperoxaluria, gout, pseudogout (chondrocalcinosis), chronic liver disease, NASH, neuroinflammation-related disorders (e.g. multiple sclerosis, brain infection, acute injury, neurodegenerative diseases, Alzheimer's disease), atherosclerosis and cardiovascular risk (e.g. cardiovascular risk reduction (CvRR), hypertension), hidradenitis suppurativa, wound healing and scar formation, and cancer (e.g. colon cancer, lung cancer, myeloproliferative neoplasms, leukemias, myelodysplastic syndromes (MDS), myelofibrosis).

In some embodiments, the compounds of formula (I), (I-a) or (I-b) or a pharmaceutically acceptable salt thereof, may be useful in the treatment of a disease or disorder mediated by NLRP3, preferably selected from a neurological disorder, symptom domains of schizophrenia and schizoaffective disorder (include negative, cognitive, depressive, positive and general psychopathology symptom domains), depression, Tourette Syndrome, Post-traumatic stress disorder (PTSD), Obsessive-compulsive disorder (OCD), analgesics, loss of memory and/or cognition associated with neurodegenerative diseases or loss of neuronal function characteristic of neurodegenerative diseases, mild cognitive impairment (MCI), Alzheimer's disease, Parkinson's disease, schizophrenia, pain, ataxia, convulsion, anxiety, traumatic brain injury (TBI), amyotrophic lateral sclerosis (ALS), multiple sclerosis (MS), fragile X syndrome (FXS), autism, or attention-deficit/hyperactivity disorder (ADHD).

In particular, the RS-D7 compound is useful in the treatment of a disease or disorder mediated by NLRP3, preferably selected from a neurological disorder, symptom domains of schizophrenia and schizoaffective disorder (include negative, cognitive, depressive, positive and general psychopathology symptom domains), depression, Tourette Syndrome, Post-traumatic stress disorder (PTSD), Obsessive-compulsive disorder (OCD), analgesics, loss of memory and/or cognition associated with neurodegenerative diseases or loss of neuronal function characteristic of neurodegenerative diseases, mild cognitive impairment (MCI), Alzheimer's disease, Parkinson's disease, schizophrenia, pain, ataxia, convulsion, anxiety, traumatic brain injury (TBI), amyotrophic lateral sclerosis (ALS), multiple sclerosis (MS), fragile X syndrome (FXS), autism, or attention-deficit/hyperactivity disorder (ADHD).

### Pharmaceutical Composition Comprising a Benzimidazole Derivative and Administration of the Benzimidazole Derivative

The pharmaceutical composition or combination of the compound used in the present disclosure can be in unit dosage of about 1-1000 mg of active ingredient(s) for a subject of about 50 - 70 kg, or about 1 - 500 mg, or about 1 - 250 mg, or about 1 - 150 mg, or about 1 - 100 mg, or about 1 - 50 mg of active ingredients. The therapeutically effective dosage of a compound, the pharmaceutical composition, or the combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The above-cited dosage properties are demonstrable in vitro and in vivo tests using advantageously mammals, e.g., mice, rats, dogs, monkeys or isolated organs, tissues and preparations thereof. The compounds of the present disclosure can be applied in vitro in the form of solutions, e.g., aqueous solutions, and in vivo either enterally, parenterally, advantageously intravenously, e.g., as a suspension or in aqueous solution. The dosage in vitro may range between about 10 3 molar and 10 9 molar concentrations. A therapeutically effective amount in vivo may range depending on the route of administration, between about 0.1 - 500 mg/kg, or between about 1 - 100 mg/kg.

Another aspect of the present disclosure provides pharmaceutical compositions which comprises a compound of Formula (I) (or a pharmaceutically acceptable salt or solvate thereof) and a pharmaceutically acceptable carrier. The term "composition", as in pharmaceutical composition, is intended to encompass a product comprising the active ingredient(s), and the inert ingredient(s) (pharmaceutically acceptable excipients) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present disclosure encompass any composition made by admixing a compound of Formula (I), additional active ingredient(s), and pharmaceutically acceptable excipients.

The pharmaceutical compositions of the present disclosure comprise a compound represented by Formula (I) (or a pharmaceutically acceptable salt or solvate thereof) as an active ingredient, a pharmaceutically acceptable carrier and optionally other therapeutic ingredients or adjuvants. The compositions include compositions suitable for oral, rectal, topical, and parenteral (including subcutaneous, intramuscular, and intravenous) administration, although the most suitable route in any given case will depend on the particular host, and nature and severity of the conditions for which the active ingredient is being administered. The pharmaceutical compositions may be conveniently presented in unit dosage form and prepared by any of the methods well known in the art of pharmacy.

The active ingredient can be administered orally in solid dosage forms, such as capsules, tablets, troches, dragées, granules and powders, or in liquid dosage forms, such as elixirs, syrups, emulsions, dispersions, and suspensions. The active ingredient can also be administered parenterally, in sterile liquid dosage forms, such as dispersions, suspensions or solutions. Other dosages forms that can also be used to administer the active ingredient as an ointment, cream, drops, transdermal patch or powder for topical administration, as an ophthalmic solution or suspension formation, i.e., eye drops, for ocular administration, as an aerosol spray or powder composition for inhalation or intranasal administration, or as a cream, ointment, spray or suppository for rectal or vaginal administration.

For topical applications, the active ingredient or a pharmaceutical composition thereof can be formulated in a suitable ointment containing the active component suspended or dissolved in one or more carriers. Carriers for topical administration of the active ingredient or a pharmaceutical composition thereof include, but are not limited to, mineral oil, liquid petrolatum, white petrolatum, propylene glycol, polyoxyethylene, polyoxypropylene compound, emulsifying wax, sugars such as lactose and water. Alternatively, the pharmaceutical compositions can be formulated in a suitable lotion or cream containing the active ingredient or a pharmaceutical composition thereof suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

Depending on the particular condition, disorder or disease to be treated, additional therapeutic agents can be administered together with the active ingredient or a pharmaceutical composition thereof. Those additional agents can be administered sequentially in any order, as part of a multiple dosage regimen, from the active ingredient or a pharmaceutical composition thereof (consecutive or intermittent administration). Alternatively, those agents can be part of a single dosage form, mixed together with the active ingredient or a pharmaceutical composition thereof (simultaneous or concurrent administration).

For oral administration, a pharmaceutical composition useful in the present disclosure can take the form of solutions, suspensions, tablets, pills, capsules, powders, granules, semisolids, sustained release formulations, elixirs, aerosols, and the like. Tablets containing various excipients such as sodium citrate, calcium carbonate and calcium phosphate are employed along with various disintegrants such as starch, preferably potato or tapioca starch, and certain complex silicates, together with binding agents such as polyvinylpyrrolidone, sucrose, gelatin and acacia. Additionally, lubricating agents such as magnesium stearate, sodium lauryl sulfate and talc are often very useful for tabletting purposes. Solid compositions of a similar type are also employed as fillers in soft and hard-filled gelatin capsules; preferred materials in this connection also include lactose or milk sugar as well as high molecular weight polyethylene glycols. When aqueous suspensions and/or elixirs are desired for oral administration, t the active ingredient or a pharmaceutical composition thereof of the present disclosure can be combined with various sweetening agents, flavoring agents, coloring agents, emulsifying agents and/or suspending agents, as well as such diluents as water, ethanol, propylene glycol, glycerin and various like combinations thereof.

The term "parenteral" as used herein refers to modes of administration which include intravenous, intramuscular, intraperitoneal, intrasternal, subcutaneous, intramedullary and intraarticular injection and infusion. A pharmaceutical composition for parenteral injection can comprise pharmaceutically acceptable sterile aqueous or nonaqueous solutions, dispersions, suspensions or emulsions as well as sterile powders for reconstitution into sterile injectable solutions or dispersions just prior to use. Aqueous solutions are especially suitable for intravenous, intramuscular, subcutaneous and intraperitoneal injection purposes. In this connection, the sterile aqueous media employed are all readily obtainable by standard techniques well-known to those skilled in the art. Examples of suitable aqueous and nonaqueous carriers, diluents, solvents or vehicles include water, ethanol, polyols (such as glycerol, propylene glycol, polyethylene glycol, and the like), carboxymethylcellulose and suitable mixtures thereof, vegetable oils (such as olive oil), and injectable organic esters such as ethyl oleate. Proper fluidity can be maintained, for example, by the use of coating materials such as lecithin, by the maintenance of the required particle size in the case of dispersions, and by the use of surfactants.

The pharmaceutical compositions useful in the present disclosure can also contain adjuvants such as, but not limited to, preservatives, wetting agents, emulsifying agents, and dispersing agents. Prevention of the action of microorganisms can be ensured by the inclusion of various antibacterial and antifungal agents, such as for example, paraben, chlorobutanol, phenol sorbic acid, and the like. It can also be desirable to include isotonic agents such as sugars, sodium chloride, and the like. Prolonged absorption of the injectable pharmaceutical form can be brought about by the inclusion of agents that delay absorption such as aluminum monostearate and gelatin.

Administration by slow infusion is particularly useful when intrathecal or epidural routes are employed. A number of implantable or body-mountable pumps useful in delivering compound at a regulated rate are known in the art.

Suspensions, in addition to the active compounds, can contain suspending agents as, for example, ethoxylated isostearyl alcohols, polyoxyethylene sorbitol and sorbitan esters, microcrystalline cellulose, aluminum metahydroxide, bentonite, agar-agar, and tragacanth, and mixtures thereof.

For purposes of transdermal (e.g., topical) administration, dilute sterile, aqueous or partially aqueous solutions (usually in about 0.1% to 5% concentration), otherwise similar to the above parenteral solutions, are prepared.

The pharmaceutical compositions useful in the present disclosure can also be administered by nasal aerosol or inhalation. Such compositions are prepared according to techniques well-known in the art of pharmaceutical formulation and can be prepared as solutions in saline, employing benzyl alcohol or other suitable preservatives, absorption promoters to enhance bioavailability, fluorocarbons, and/or other conventional solubilizing or dispersing agents.

Compositions for rectal or vaginal administration are preferably suppositories which can be prepared by mixing the active ingredient or a pharmaceutical composition thereof with suitable non-irritating excipients or carriers such as cocoa butter, polyethylene glycol or a suppository wax which are solid at room temperature but liquid at body temperature and therefore melt in the rectum or vaginal cavity and release the drugs.

Other pharmaceutically acceptable carriers include, but are not limited to, a non-toxic solid, semisolid or liquid filler, diluent, encapsulating material or formulation auxiliary of any type, including but not limited to ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

Solid pharmaceutical excipients include, but are not limited to, starch, cellulose, talc, glucose, lactose, sucrose, gelatin, malt, rice, flour, chalk, silica gel, magnesium stearate, sodium stearate, glycerol monostearate, sodium chloride, dried skim milk and the like. Liquid and semisolid excipients can be selected from glycerol, propylene glycol, water, ethanol and various oils, including those of petroleum, animal, vegetable or synthetic origin, e.g., peanut oil, soybean oil, mineral oil, sesame oil, etc. Preferred liquid carriers, particularly for injectable solutions, include water, saline, aqueous dextrose, and glycols.

Methods of preparing various pharmaceutical compositions with a certain amount of active ingredient are known, or will be apparent in light of this disclosure, to those skilled in this art. Other suitable pharmaceutical excipients and their formulations are described in Remington's Pharmaceutical Sciences, edited by E. W. Martin, Mack Publishing Company, 19th ed. 1995.

Without further elaboration, it is believed that one skilled in the art can utilize the present disclosure to its fullest extent on the basis of the preceding description. The following examples are, therefore, to be construed as merely illustrative and not a limitation of the scope of the present disclosure in any way.

The present invention further relates to the following items:
1. A method for prevention and/or treatment of a disease or disorder mediated by NLRP3 in a subject, comprising administering an effective amount of a compound of Formula (I) or a pharmaceutically acceptable salt, solvate or prodrug as an active ingredient to the subject, 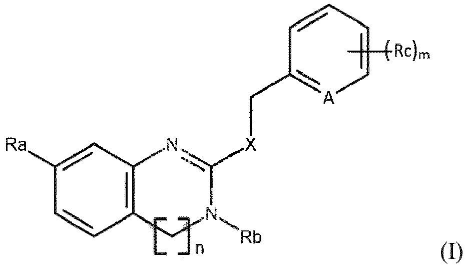
   wherein n is 0 or 1,
   X is -S-, -S(=O)- or -NRₙ-; wherein
      Rₙ is H or 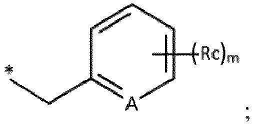
   A is -CH, -CR_{c} or N;
   Rₐ is -C(=O)ORₐ₁, -ORₐ₂, -O-C(=O)Rₐ₃ or -O-C(=O)-T-ORₐ₄; wherein
      Rₐ₁ is H or linear or branched C₁₋₁₅alkyl;
      Rₐ₂ is H, linear or branched C₁₋₁₅alkyl, phosphonate, diarylphosphonate or an *O-*protecting group;
      Rₐ₃ and Rₐ₄ are independently a protecting group, linear or branched C₁₋₁₅alkyl, linear or branched C₁₋₁₅alkenyl, -T-C₃₋₁₀cycloalkyl, -T-NHRₐ₃ₚ, -T-C₃₋₁₀cycloalkenyl, -T-C₆₋₁₀aryl, -T-C₅₋₁₀heteroaryl, -T-NH-C(=O)-O-C₁₋₁₀alkyl, -T-adamantyl or -C₁₋₃alkylene-C₆₋₁₀aryl where the alkylene is substituted with -T-NHRₐ₃ₚ;
      Rₐ₃ₚ is H or an *N*-protecting group;
   R_{b} is H, linear or branched C₁₋₁₅alkyl, linear or branched C₂₋₁₅alkenyl, C₁₋₃alkoxy-C₁₋₁₅alkyl-, -T'-C₃₋₁₀cycloalkyl, -T'-C₃₋₁₀cycloalkenyl, -T'-C₆₋₁₀ aryl or -T'-C₅₋₁₀heteroaryl;
   R_{c} each is independently halogen, linear or branched C₁₋₁₅alkyl, linear or branched C₁₋₁₅alkoxyl, unprotected or protected hydroxyl group, or -C₁₋₁₀alkylene-Y-C₆₋₁₀heteroaryl wherein -Y- is -CH₂-, -NH-, -O- or -S-;
   symbol ^{∗} represents the bonding position;
   m is an integer from 0 to 4;
   -T- is absent, C₁₋₃alkylene or C₂₋₃alkenylene;
   -T'- is C₁₋₃alkylene or C₂₋₃alkenylene; and
   wherein the heteroaryl contains at least one heteroatom, each heteroatom being independently S, N or O;
   wherein the alkyl, alkenyl, alkoxy, cycloalkyl, aryl, heteroaryl, alkylene and alkenylene are each independently unsubstituted or substituted with at least one substituent;
   wherein the substituent is each independently a halogen, a protecting group, protected or unprotected amino group, nitro, nitroso, linear or branched C₁₋₁₅ alkyl,or linear or branched C₁₋₁₅ alkoxy or C₃₋₁₀cycloalkyl; and
   when R_{b} is H, the tautomers are included,
   with the proviso that
   when X is -S- or -S(=O)-, Rₐ is -ORₐ₂ and Rₐ₂ is H or linear or branched C₁₋₁₅alkyl, then A is -CH or -CR_{c};
   when X is -S- or -S(=O)- and Rₐ is -C(=O)ORₐ₁, R_{b} is linear or branched C₆₋₁₅alkyl, linear or branched C₆₋₁₅alkenyl, C₁₋₃alkoxy-C₁₋₁₅alkyl-, -T'-C₃₋₁₀cycloalkyl, -T'-C₃₋₁₀cycloalkenyl, - T'-C₆₋₁₀ aryl or -T'-C₅₋₁₀heteroaryl;
   or a pharmaceutically acceptable salt thereof.
2. The method of item 1, wherein the disease or disorder is selected from inflammasome-related diseases or disorders, immune diseases, inflammatory diseases, auto-immune diseases, or auto-inflammatory diseases, liver related diseases or disorders, inflammatory arthritis related disorders, kidney related diseases, neuroinflammation-related diseases, cardiovascular or metabolic diseases or disorders, inflammatory skin diseases, wound healing and scar formation, asthma, sarcoidosis, age- related macular degeneration, or cancer related diseases or disorders; or the disease or disorder is selected from autoinflammatory fever syndromes, chronic liver disease, viral hepatitis, non alcoholic steatohepatitis (NASH), alcoholic steatohepatitis, alcoholic liver disease, gout, pseudogout (chondrocalcinosis), osteoarthritis, rheumatoid arthritis, arthropathy, hyperoxaluria, lupus nephritis, hypertensive nephropathy, hemodialysis related inflammation, multiple sclerosis, brain infection, acute injury, neurodegenerative diseases, Alzheimer's disease (AD), cardiovascular risk reduction (CvRR), hypertension, atherosclerosis, type I and type II diabetes and related complications, peripheral artery disease (PAD), acute heart failure, hidradenitis suppurativa, acne, colon cancer, lung cancer, myeloproliferative neoplasms, leukemias, myelodysplastic syndromes (MDS), myelofibrosis, multiple system atrophy (MSA), or dementia with Lewy bodies; or the disease or disorder is selected from cryopyrin-associated periodic syndrome, nephropathy, or retinopathy; or the disease or disorder is selected from a neurological disorder, symptom domains of schizophrenia and schizoaffective disorder, depression, Tourette Syndrome, Post-traumatic stress disorder (PTSD), Obsessive-compulsive disorder (OCD), analgesics, loss of memory and/or cognition associated with neurodegenerative diseases or loss of neuronal function characteristic of neurodegenerative diseases, mild cognitive impairment (MCI), Alzheimer's disease, Parkinson's disease (PD), schizophrenia, pain, ataxia, convulsion, anxiety, traumatic brain injury (TBI), amyotrophic lateral sclerosis (ALS), multiple sclerosis (MS), fragile X syndrome (FXS), autism, or attention-deficit/hyperactivity disorder (ADHD).
3. The method of item 1, wherein n is 0.
4. The method of item 1, wherein m is an integer from 0 to 3.
5. The method of item 1, wherein:
   n is 0;
   X is -S(=O);
   A is N;
   Rₐ is -O-C(=O)Rₐ₃ or -O-C(=O)-T-ORₐ₄; wherein
      Rₐ₃ is a linear or branched C₁₋₁₅alkyl, linear or branched C₂₋₁₅alkenyl, -T-C₃₋₁₀cycloalkyl, -T-NHRₐ₃ₚ, -T-C₃₋₁₀cycloalkenyl, -T-C₆₋₁₀aryl, -T-C₅₋₁₀heteroaryl, -T-NH-C(=O)-O-C₁₋₁₀alkyl, -T-adamantyl or -C₁₋₃alkylene-C₆₋₁₀aryl where the alkylene is substituted with -T-NHRₐ₃ₚ;
      Rₐ₄ is a linear or branched C₁₋₁₅alkyl, linear or branched C₂₋₁₅alkenyl, -T-C₃₋₁₀cycloalkyl, -T-NHRₐ₃ₚ, -T-C₃₋₁₀cycloalkenyl, -T-C₆₋₁₀aryl, -T-C₅₋₁₀heteroaryl, -T-NH-C(=O)-O-C₁₋₁₀alkyl, -T-adamantyl or -C₁₋₃alkylene-C₆₋₁₀aryl where the alkylene is substituted with -T-NHRₐ₃ₚ;
      Rₐ₃ₚ is H or an *N*-protecting group selected from Fmoc or Boc;
   or a pharmaceutically acceptable salt thereof.
6. The method of item 5, wherein:
   A is N;
   R_{b} is H;
   m is 3; and
   R_{c} each is independently linear or branched C₁₋₁₅alkyl, linear or branched C₁₋₁₅alkoxyl; or a pharmaceutically acceptable salt.
7. The method of item 5, wherein Rₐ is -O-C(=O)Rₐ₃ wherein Rₐ₃ is adamantyl; linear or branched C₁₋₁₀alkyl unsubstituted or substituted by halogen; C₁₋₄alkoxy; -C₆₋₁₀aryl unsubstituted or substituted by C₁₋₁₀ alkyl, nitro, C₁₋₁₅alkoxy or halogen; C₃₋₁₀cycloalkyl; -C₃₋₁₀cycloalkenyl; linear or branched C₂₋₁₀alkenyl; -C₅₋₁₀heteroaryl; -C₁₋₃alkylene-C₃₋₁₀cycloalkyl; C₂₋₃alkenylene-C₆₋₁₀aryl wherein C₆₋₁₀aryl is unsubstituted or substituted by halogen; -O-C(=O)-O-C₁₋₁₀alkyl.
8. The method of item 5, wherein Rₐ is -O-C(=O)Rₐ₃ wherein Rₐ₃ is adamantyl; linear or branched C₁₋₈alkyl unsubstituted or substituted by halogen; C₁₋₄alkoxy; -phenyl unsubstituted or substituted by C₁₋₆ alkyl, nitro, C₁₋₄alkoxy or halogen; C₃₋₆cycloalkyl; -C₃₋₆cycloalkenyl; linear or branched C₂₋₆alkenyl; -C₅₋₆heteroaryl; -C₁₋₃alkylene-C₃₋₆cycloalkyl; C₂₋₃alkenylene-phenyl wherein phenyl is unsubstituted or substituted by halogen; -O-C(=O)-O-C₁₋₄alkyl.
9. The method of item 5, wherein Rₐ is -O-C(=O)-C₁₋₆alkyl, -O-C(=O)-C₁₋₃alkylene-NHFmoc,-O-C(=O)-C₁₋₃alkylene-NHBoc or -O-C(=O)-NH-C(=O)-O-C₁₋₁₀alkyl.
10. The method of item 5, wherein R_{c} each is independently halogen, linear or branched C₁₋₆alkyl, linear or branched C₁₋₆alkoxyl, or -C₁₋₁₀alkenylene-Y-C₆₋₁₀heteroaryl; wherein Y is S and C₆₋₁₀heteroaryl is unsubstituted or substituted by C₁₋₁₅alkyl, C₁₋₁₅alkoxy, -OH, -NH₂, -NO₂ or halogen.
11. The method of item 1, wherein:
   X is -S-;
   A is N;
   Rₐ is -C(=O)ORₐ₁; wherein Rₐ₁ is H or linear or branched C₁₋₁₅alkyl;
   or a pharmaceutically acceptable salt thereof.
12. The method of item 11, wherein Rₐ is -C(=O)OH, or -C(=O)OC₁₋₄alkyl.
13. The method of item 1, wherein the compound is selected from the group consisting of:
   RS-D7: 5-O-Desmethyl-Omeprazole
   Esomeprazole: (S)-(-)-5-Methoxy-2-[(4-methoxy-3,5-dimethylpyridin-2-yl)methylsulfinyl]-3H-benzoimidazole
   21122: (2-(((4-methoxy-3,5-dimethylpyridin-2-yl)methyl)sulfinyl)-1H-benzo[d]imidazol-5-yl acetate)
   21124: (2-(((4-methoxy-3,5-dimethylpyridin-2-yl)methyl)sulfinyl)-1H-benzo[d]imidazol-5-yl benzoate)
   26096: (2-(((4-methoxy-3,5-dimethylpyridin-2-yl)methyl)sulfinyl)-1H-benzo[d]imidazol-5-yl butyrate)
   26097: (2-(((4-methoxy-3,5-dimethylpyridin-2-yl)methyl)sulfinyl)-1H-benzo[d]imidazol-5-yl cyclohexanecarboxylate)
   26098: (2-(((4-methoxy-3,5-dimethylpyridin-2-yl)methyl)sulfinyl)-1H-benzo[d]imidazol-5-yl 4-butylbenzoate)
   21127: (2-(((4-methoxy-3,5-dimethylpyridin-2-yl)methyl)sulfinyl)-1H-benzo[d]imidazol-5-yl 3-methylbenzoate)
   27076: 2-(((4-methoxy-3,5-dimethylpyridin-2-yl)methyl)sulfinyl)-1H-benzo[d]imidazol-5-yl hexanoate
   27077: 2-(((4-methoxy-3,5-dimethylpyridin-2-yl)methyl)sulfinyl)-1H-benzo[d]imidazol-5-yl isobutyrate
   27078: (2-(((4-methoxy-3,5-dimethylpyridin-2-yl)methyl)sulfinyl)-1H-benzo[d]imidazol-5-yl cyclohex-3-ene-1-carboxylate)
   27079: 2-(((4-methoxy-3,5-dimethylpyridin-2-yl)methyl)sulfinyl)-1H-benzo[d]imidazol-5-yl cyclohex-3-enecarboxylate
   28087: (2-(((4-methoxy-3,5-dimethylpyridin-2-yl)methyl)sulfinyl)-1H-benzo[d]imidazol-5-yl 4-methylbenzoate)
   28091: (2-(((4-methoxy-3,5-dimethylpyridin-2-yl)methyl)sulfinyl)-1H-benzo[d]imidazol-5-yl 2-nitrobenzoate)
   28092: (2-(((4-methoxy-3,5-dimethylpyridin-2-yl)methyl)sulfinyl)-1H-benzo[d]imidazol-5-yl cyclopropanecarboxylate)
   28093: (2-(((4-methoxy-3,5-dimethylpyridin-2-yl)methyl)sulfinyl)-1H-benzo[d]imidazol-5-yl 2-ethylbutanoate)
   28094: (2-(((4-methoxy-3,5-dimethylpyridin-2-yl)methyl)sulfinyl)-1H-benzo[d]imidazol-5-yl 2-phenylacetate)
   28095: (2-(((4-methoxy-3,5-dimethylpyridin-2-yl)methyl)sulfinyl)-1H-benzo[d]imidazol-5-yl 3,5,5-trimethylhexanoate
   28096: (2-(((5-methoxy-4,6-dimethylpyridin-2-yl)methyl)sulfinyl)-1H-benzo[d]imidazol-5-yl 2-ethoxybenzoate)
   21123: (2-(((4-methoxy-3,5-dimethylpyridin-2-yl)methyl)sulfinyl)-1H-benzo[d]imidazol-5-yl propionate)
   21125: (2-(((4-methoxy-3,5-dimethylpyridin-2-yl)methyl)sulfinyl)-1H-benzo[d]imidazol-5-yl 4-chlorobenzoate)
   21126: (2-(((4-methoxy-3,5-dimethylpyridin-2-yl)methyl)sulfinyl)-1H-benzo[d]imidazol-5-yl 3-nitrobenzoate)
   21128: 2-(((4-methoxy-3,5-dimethylpyridin-2-yl)methyl)sulfinyl)-1H-benzo[d]imidazol-5-yl heptanoate
   21129: (2-(((4-methoxy-3,5-dimethylpyridin-2-yl)methyl)sulfinyl)-1H-benzo[d]imidazol-5-yl 4-fluorobenzoate)
   21130: (2-(((4-methoxy-3,5-dimethylpyridin-2-yl)methyl)sulfinyl)-1H-benzo[d]imidazol-5-yl (Z)-2-methylbut-2-enoate)
   21131: (2-(((4-methoxy-3,5-dimethylpyridin-2-yl)methyl)sulfinyl)-1H-benzo[d]imidazol-5-yl 2-chloropropanoate)
   21132: tert-butyl (2-(((4-methoxy-3,5-dimethylpyridin-2-yl)methyl)sulfinyl)-1H-benzo[d]imidazol-5-yl) carbonate
   12124: (2-(((4-methoxy-3,5-dimethylpyridin-2-yl)methyl)sulfinyl)-1H-benzo[d]imidazol-5-yl (Z)-but-2-enoate)
   12125: (2-(((4-methoxy-3,5-dimethylpyridin-2-yl)methyl)sulfinyl)-1H-benzo[d]imidazol-5-yl 3-methylbut-2-enoate)
   12122: (2-(((4-methoxy-3,5-dimethylpyridin-2-yl)methyl)sulfinyl)-1H-benzo[d]imidazol-5-yl furan-2-carboxylate)
   12123: (2-(((4-methoxy-3,5-dimethylpyridin-2-yl)methyl)sulfinyl)-1H-benzo[d]imidazol-5-yl acrylate)
   12127: (2-(((4-methoxy-3,5-dimethylpyridin-2-yl)methyl)sulfinyl)-1H-benzo[d]imidazol-5-yl 2-methylbutanoate)
   12128: (2-(((4-methoxy-3,5-dimethylpyridin-2-yl)methyl)sulfinyl)-1H-benzo[d]imidazol-5-yl 3-cyclopentylpropanoate)
   12129: (2-(((4-methoxy-3,5-dimethylpyridin-2-yl)methyl)sulfinyl)-1H-benzo[d]imidazol-5-yl (E)-3-(2-chlorophenyl)acrylate)
   12130: (2-(((4-methoxy-3,5-dimethylpyridin-2-yl)methyl)sulfinyl)-1H-benzo[d]imidazol-5-yl 6-bromohexanoate)
   11021: (2-(((4-methoxy-3,5-dimethylpyridin-2-yl)methyl)sulfinyl)-1H-benzo[d]imidazol-5-yl 2-fluorobenzoate)
   11020: (2-(((4-methoxy-3,5-dimethylpyridin-2-yl)methyl)sulfinyl)-1H-benzo[d]imidazol-5-yl 4-methoxybenzoate)
   11022: (2-(((4-methoxy-3,5-dimethylpyridin-2-yl)methyl)sulfinyl)-1H-benzo[d]imidazol-5-yl (3r,5r,7r)-adamantane-1-carboxylate)
   11023: (2-(((4-methoxy-3,5-dimethylpyridin-2-yl)methyl)sulfinyl)-1H-benzo[d]imidazol-5-yl isoxazole-5-carboxylate)
   11030: (2-(((4-methoxy-3,5-dimethylpyridin-2-yl)methyl)sulfinyl)-1H-benzo[d]imidazol-5-yl 4-(tert-butyl)benzoate)
   11031: (2-(((4-methoxy-3,5-dimethylpyridin-2-yl)methyl)sulfinyl)-1H-benzo[d]imidazol-5-yl 3-chloro-4-fluorobenzoate)
   25015: (2-(((4-methoxy-3,5-dimethylpyridin-2-yl)methyl)sulfinyl)-1H-benzo[d]imidazol-5-yl pivalate)
   25016: (2-(((4-methoxy-3,5-dimethylpyridin-2-yl)methyl)sulfinyl)-1H-benzo[d]imidazol-5-yl pentanoate)
   25017: (2-(((4-methoxy-3,5-dimethylpyridin-2-yl)methyl)sulfinyl)-1H-benzo[d]imidazol-5-yl 4-nitrobenzoate)
   25027: (2-(((4-methoxy-3,5-dimethylpyridin-2-yl)methyl)sulfinyl)-1H-benzo[d]imidazol-5-yl cyclobutanecarboxylate)
   25028: (2-(((4-methoxy-3,5-dimethylpyridin-2-yl)methyl)sulfinyl)-1H-benzo[d]imidazol-5-yl thiophene-2-carboxylate)
   25029: (2-(((4-methoxy-3,5-dimethylpyridin-2-yl)methyl)sulfinyl)-1H-benzo[d]imidazol-5-yl 2-methylbutanoate)
   25030: (2-(((4-methoxy-3,5-dimethylpyridin-2-yl)methyl)sulfinyl)-1H-benzo[d]imidazol-5-yl 3,3-dimethylbutanoate)
   25031: (2-(((4-methoxy-3,5-dimethylpyridin-2-yl)methyl)sulfinyl)-1H-benzo[d]imidazol-5-yl 2-methoxyacetate) and
   25032: (ethyl (2-(((4-methoxy-3,5-dimethylpyridin-2-yl)methyl)sulfinyl)-1H-benzo[d]imidazol-5-yl) carbonate)
   or a pharmaceutically acceptable salt thereof.
5. The method of Claim 1, wherein the compound is selected from the group consisting of:
   12082: Methyl 1-(2-(cyclohex-1-en-1-yl)ethyl)-2-(((4-methoxy-3,5-dimethylpyridin-2-yl)methyl)thio)-1H-benzo[d]imidazole-5-carboxylate
   12083: 1-(2-(cyclohex-1-en-1-yl)ethyl)-2-(((4-methoxy-3,5-dimethylpyridin-2-yl)methyl)thio)-1H-benzo[d]imidazole-5-carboxylic acid
   12084: Methyl 1-(2-(cyclohex-1-en-1-yl)ethyl)-2-(((4-methoxy-3,5-dimethylpyridin-2-yl)methyl)sulfinyl)-1H-benzo[d]imidazole-5-carboxylate
   12088: methyl 2-(((4-methoxy-3,5-dimethylpyridin-2-yl)methyl)thio)-1-octyl-1H-benzo[d]imidazole-5-carboxylate
   21098: Methyl 2-(((4-methoxy-3,5-dimethylpyridin-2-yl)methyl)amino)- 1-propyl-1H-benzo[d]imidazole-5-carboxylate
   26065: Methyl 1-(furan-2-ylmethyl)-2-(((4-methoxy-3,5-dimethylpyridin-2-yl)methyl)amino)-1H-benzo[d]imidazole-5-carboxylate
   21102: 2-(((4-methoxy-3,5-dimethylpyridin-2-yl)methyl)amino)-1-propyl- 1H-benzo[d]imidazole-5-carboxylic acid
   21103: Methyl 2-(((4-methoxy-3,5-dimethylpyridin-2-yl)methyl)amino)-1- (3-methoxypropyl)-1H-benzo[d]imidazole-5-carboxylate
   21104: 2-(bis((4-methoxy-3,5-dimethylpyridin-2-yl)methyl)amino)-1- (3-methoxypropyl)-1H-benzo[d]imidazole-5-carboxylic acid
   26066: Methyl 2-(((4-methoxy-3,5-dimethylpyridin-2-yl)methyl)amino)-1-pentyl-1H-benzo[d]imidazole-5-carboxylate
   21105: 2-(bis((4-methoxy-3,5-dimethylpyridin-2-yl)methyl)amino)-1- (2-(cyclohex-1-en-1-yl)ethyl)-1H-benzo[d]imidazole-5-carboxylic acid
   26070: 1-(furan-2-ylmethyl)-2-(((4-methoxy-3,5-dimethylpyridin-2-yl)methyl)amino)-1H-benzo[d]imidazole-5-carboxylic acid
   26071: Methyl 2-(((4-methoxy-3,5-dimethylpyridin-2-yl)methyl)amino)-1-(4-methoxybenzyl)-1H-benzo[d]imidazole-5-carboxylate
   26072: Methyl 2-(bis((4-methoxy-3,5-dimethylpyridin-2-yl)methyl)amino)-3-(furan-2-ylmethyl)-3,4-dihydroquinazoline-7-carboxylate
   21106: Methyl 3-(2-(cyclohex-1-en-1-yl)ethyl)-2-(((4-methoxy-3,5- dimethylpyridin-2-yl)methyl)amino)-3,4-dihydroquinazoline-7-carboxylate
   12092: 2-(((4-methoxy-3,5-dimethylpyridin-2-yl)methyl)thio)-1H-benzo[d]imidazol-5-yl (((9H-fluoren-9-yl)methoxy)carbonyl)glycinate
   12093: 2-(((4-methoxy-3,5-dimethylpyridin-2-yl)methyl)thio)-1H-benzo[d]imidazol-5-yl (tert-butoxycarbonyl)glycinate
   21110: 2-(((4-methoxy-3,5-dimethylpyridin-2-yl)methyl)amino)-3- (3-methoxypropyl)-3,4-dihydroquinazoline-7-carboxylic acid
   26076: 2-(((4-methoxy-3,5-dimethylpyridin-2-yl)methyl)amino)-1-pentyl-1H-benzo[d]imidazole-5-carboxylic acid
   26077: 2-(((4-methoxy-3,5-dimethylpyridin-2-yl)methyl)amino)-1-(4-methoxybenzyl)-1H-benzo[d]imidazole-5-carboxylic acid
   12094: 2-(((4-methoxy-3,5-dimethylpyridin-2-yl)methyl)thio)-1H benzo[d]-imidazole-5-yl (S)-2-((((9H-fluoren-9-yl)methoxy)carbonyl)amino)-2-phenylacetate
   13001: 2-(((4-methoxy-3,5-dimethylpyridin-2-yl)methyl)sulfinyl)-1H-benzo[d]imidazol-6-yl 2-((tert-butoxycarbonyl)amino)acetate
   13084: 2-(((4-methoxy-3,5-dimethylpyridin-2-yl)methyl)sulfinyl)-1H-benzo[d]imidazol-5-yl diphenyl phosphate
   26079: Methyl 3-(furan-2-ylmethyl)-2-(((4-methoxy-3,5-dimethylpyridin-2-yl)methyl)amino)-3,4-dihydroquinazoline-7-carboxylate
   26089: 3-(furan-2-ylmethyl)-2-(((4-methoxy-3,5-dimethylpyridin-2-yl)methyl)amino)-3,4-dihydroquinazoline-7-carboxylic acid
   26090: 2-(((4-methoxy-3,5-dimethylpyridin-2-yl)methyl)amino)-3-pentyl-3,4-dihydroquinazoline-7-carboxylic acid
   26091: Methyl 2-(((4-methoxy-3,5-dimethylpyridin-2-yl)methyl)amino)-3-pentyl-3,4-dihydroquinazoline-7-carboxylate
   26092: Methyl 2-(((4-methoxy-3,5-dimethylpyridin-2-yl)methyl)amino)-3-(4-methoxybenzyl)-3,4-dihydroquinazoline-7-carboxylate
   21115: 2-(((4-methoxy-3,5-dimethylpyridin-2-yl)methyl)amino)-1- (3-methoxypropyl)-1H-benzo[d]imidazole-5-carboxylic acid
   21116: Methyl 1-(2-(cyclohex-1-en-1-yl)ethyl)-2-(((4-methoxy-3,5- dimethylpyridin-2-yl)methyl)amino)-1H-benzo[d]imidazole-5-carboxylate
   21117: 1-(2-(cyclohex-1-en-1-yl)ethyl)-2-(((4-methoxy-3,5-dimethylpyridin-2-yl)methyl)amino)-1H-benzo[d]imidazole-5-carboxylic acid
   21118: Methyl 2-(((4-methoxy-3,5-dimethylpyridin-2-yl)methyl)amino)-1- phenethyl-1H-benzo[d]imidazole-5-carboxylate
   21119: 2-(((4-methoxy-3,5-dimethylpyridin-2-yl)methyl)amino)-1-phenethyl- 1H-benzo[d]imidazole-5-carboxylic acid
   21120: 2-(bis((4-methoxy-3,5-dimethylpyridin-2-yl)methyl)amino)-1-propyl- 1H-benzo[d]imidazole-5-carboxylic acid
   21121: 2-(bis((4-methoxy-3,5-dimethylpyridin-2-yl)methyl)amino)-1-phenethyl- 1H-benzo[d]imidazole-5-carboxylic acid
   22138: 5-methoxy-2-((2-methoxy-3,6-dimethylbenzyl)thio)-1H-benzo[d]imidazole
   22139: 5-methoxy-2-((2-methoxy-3,6-dimethylbenzyl)sulfinyl)-1H-benzo[d]imidazole
   22140: 2,2'-(((2-methoxy-4-methyl-1,3-phenylene)bis(methylene))bis(sulfanediyl))bis(5-methoxy-1H-benzo[d]imidazole)
   21133: 2-((3-(bromomethyl)-2-((tert-butyldimethylsilyl)oxy)-6-methylbenzyl)thio)-5-methoxy-1H-benzo[d]imidazole and
   22141: 2-((2-methoxy-3,6-dimethylbenzyl)thio)-1H-benzo[d]imidazol-5-ol or a pharmaceutically acceptable salt thereof.
14. The method of item 12, wherein R_{c} each is independently halogen, linear or branched C₁₋₆alkyl, linear or branched C₁₋₆alkoxyl, or -C₁₋₁₀alkenylene-Y-C₆₋₁₀heteroaryl; wherein Y is S and C₆₋₁₀heteroaryl is unsubstituted or substituted by C₁₋₁₅alkyl, C₂₋₁₅alkenyl, C₁₋₁₅alkoxy, -OH, - NH2, -NO₂ or halogen.

### Examples

### Example 1 NLRP3 inflammasome assay

The pathogen-associated molecular pattern (PAMP) lipopolysaccharide (LPS) and the danger-associated molecular patterns (DAMP) including monosodium urate crystals (MSU) or Adenosine triphosphate (ATP) are stimuli used to activate the NLRP3 inflammasome, which then triggers a series of downstream events, including the activation of caspase-1 and the subsequent conversion of pro-IL-1β to pro-inflammatory cytokine IL-1β. The assays were conducted using Human promonocytic leukemia THP-1 cells.

### Methods

Human promonocytic leukemia THP-1 cells were cultured in RPMI and treated with *Phorbol 12-myristate 13-acetate* (PMA). PMA-primed THP-1 cells were treated with 50 ng/mL LPS for 2 hours, and then pre-treated with the diarylsulfonylurea MCC950 (1uM) (*Nature Medicine. **21** (3): 248*-*55, 2015),* RS-D7 (0.1, 1, and 5 uM), 12083 (0.2, 1, and 5 uM), 12127 (0.2, 1, and 5 uM), 28093 (0.2, 1, and 5 uM), or 12093 (0.2, 1, and 5 uM) for 1 hour; and then stimulated with 5mM ATP for 20 minutes or 400 ug/ml MSU for 5 hours. Culture media was placed with MCC950, RS-D7, 12083, 12127, 28093, or 12093-containing RPMI medium and cultured for 2 hours (This step was only for the ATP as the activator). The supernatant of PMA-differentiated THP-1 cells was collected at given time points after stimulation, and IL-1β level in cell culture supernatant was determined using IL-1 beta Human Uncoated ELISA Kit (Invitrogen). Supernatants and cell lysate were also evaluated by immunoblotting methods. The immunoreactive signals were developed using Immobilon Crescendo Western HRP substrate and the digitized image of the membrane was detected by ImageQuant LAS 4000mini (GE) according to the manufacturer's instructions.

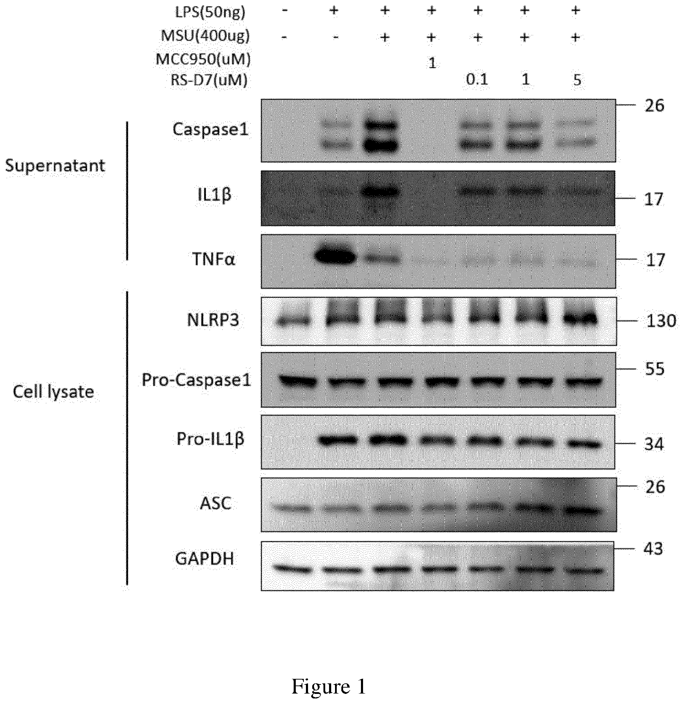

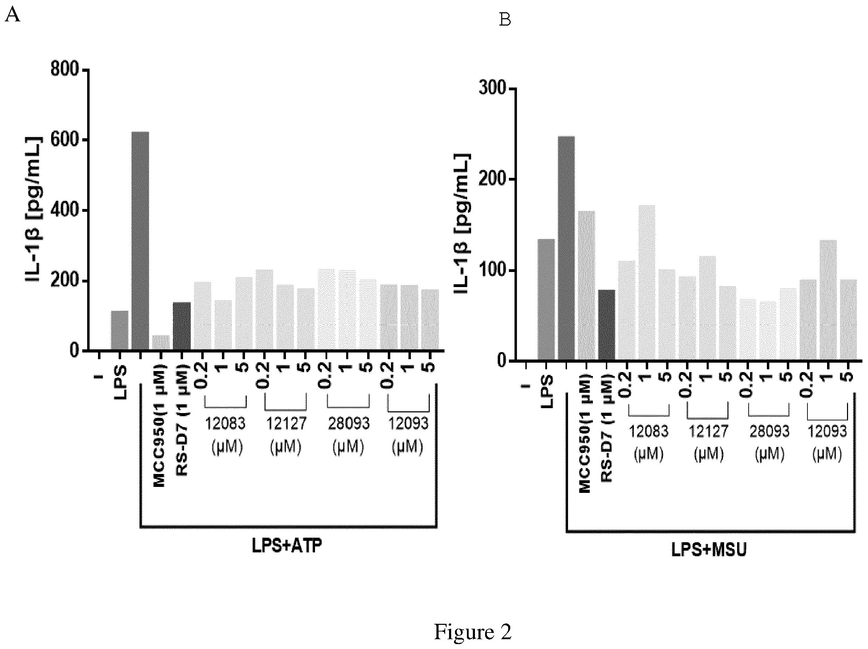

The western blot in **Figure 1** shows LPS+MSU led to increased caspase-1 and IL-1β. MCC950, an NLRP3 inhibitor (positive control), inhibited the expression of caspase-1 and IL-1β. Treatment with RS-D7 showed dose-dependent inhibition of caspase-1 and IL-1β. As no individual protein was inhibited by RS-D7, it is speculated that RS-D7 may inhibit the process of NLRP3 inflammasome assembly from driving a reduction in IL-1β.
The ELISA result in **Figure 2** shows that in LPS- and ATP-treated cells, 600 pg/mL of IL-1β was released. A significantly lower level of IL-1β was detected in cells treated with MCC950. Compared to the LPS+ATP group or LPS+MSU group, significantly lower levels of IL-1β were observed in cells treated with RS-D7, and different concentrations of 12083, 12127, 28093, and 12093. The reduction in IL-1β suggests these compounds are effective NLRP3 inflammasome modulators.

### Example 2 In vivo efficacy of RS-D7 in TgM83 mouse model of neuronal alpha-synucleinopathies related neurodegenerative diseases

### Method

### 2.1 Animal information

Male TgM83 and their wild-type (WT) littermate mice were used in this example. The TgM83 mice homozygous for the human A53T-α-Synuclein mutation driven by the prion promoter (A53T) were obtained from the Jackson Laboratory (Bar Harbor, ME, United States, https://www.jax.org/strain/004479) and they were generated from A53T-heterozygous breeding pairs in a mixed C57BL/6J × C3H background to produce transgenic homozygous (B6;C3-Tg(Prnp-SNCA^{∗}A53T)83Vle/J, which is known as TgM83; *A53T*^{+/+}) and nontransgenic (WT; *A53T*^{-/-}) littermates (Giasson et al., 2002). The animals were also genotyped using PCR analysis (from mouse-tail DNA), as described previously (Graham & Sidhu, 2010). The experiments were performed on the TgM83 mice aged 12-month-old.

### 2.2 Animal Welfare Compliance and husbandry

All animal procedures were performed according to the protocols approved by the Animal Care and Use Committee of National Taiwan University. All animals used in this example were housed with food and water available ad libitum in ventilated polysulfone cages (Alternative design Inc., Winthrop, Maine, USA) within the animal rooms of the Psychology Department of National Taiwan University. Rooms were maintained at 22 ± 2 °C and kept on a 12:12 light/dark cycle (lights on at 05:00). Animals were housed individually at least one week before experimental testing, and all behavioral and pharmacological tasks were conducted during the dark cycle. The minimum number of animals were used to meet the 3R reduction principle of animal use. Adequate measures were taken to minimize potential pain or discomfort that the animal use in this example may have experienced.

### Dose formulations

The mice were dosed with a constant volume of 10 mL/kg body weight. RS-D7 was freshly prepared before the experiment, and then discarded after use in the target animals.

### 2.3 Experimental design and behavioral tasks

Mice used in this example were divided into three groups including a) WT littermate mice with vehicle control group (WT control), b) TgM83 mice with vehicle control group (TgM83 mice), and c) TgM83 mice with RS-D7 group (TgM83 + RS-D7 30 mg/kg (PO)). The number of animals in each group was 10. All animals were given a single dose of the vehicle (ddH₂O, P.O.) or RS-D7 (30 mg/kg, P.O.) 20 minutes before the behavioral experiment started. The open field task was performed in the first week, and the rotarod task was performed in the second week. The details of each of the behavioral tasks have been described as follows.

### 2.4 Open field task

To assess spontaneous locomotor activity, each animal was placed into the center of an open field apparatus (48 cm × 24 cm × 25 cm, Coulbourn Instruments, White hall, PA, USA) under dim lighting condition (60 lx). Motor activity parameter (total travel distance moved) were monitored and recorded for 1-hour period using the SMART video tracking system (San Diego Instruments, San Diego, CA).

### 2.5 Rotarod task

Mice were tested for motor coordination and motor skill learning as described previously (Brooks, Trueman, & Dunnett, 2012). On the training day, mice were trained for 5 min at a constant speed of 4 rpm on the rotarod apparatus (LE8200, PanLab) three times. Twenty-four hours after training, mice were tested on the rotarod at an accelerating speed (4 - 40 rpm within 5 min). Three testing trials were performed on the testing day. The latency of a mouse falling off the rotating rod was recorded automatically by the stop-plates.

### 2.6 Examination of the inflammation cytokines treating with RS-D7 in TgM83 mouse model

To characterize the inflammatory response after RS-D7 treatment, the level of inflammation cytokines, including interleukin-1 beta (IL-1β), interleukin-6 (IL-6), and Tumor Necrosis Factor-a (TNF-α), were examined in the cerebellum of male TgM83 and WT littermate mice (n = 5 each) using enzyme-linked immunosorbent assay (ELISA). All the animals were sacrificed after 20-minutes RS-D7 treatment and the tissue samples were taken from hippocampus of the mouse brain from male WT littermates, TgM83 mice and TgM83 mice with RS-D7 treatment (30 mg/kg, P.O.). These samples were dissected on ice and then stored at -80°C before use. The ELISA experiment was performed using commercial kits for IL-1β (88-7013, Invitrogen, USA), IL-6 (88-7064, Invitrogen, USA) and TNF-α (88-7324, Invitrogen, USA). Procedures were followed according to manufactures protocols. Standards and samples were run in triplicates. OD values were read at 450 nm using a microplate reader (ThermoFisher Scientific, USA). Calculation of concentrations were done according to manufactures protocol using standard curve methods.

### 2.7 Statistical analysis

All of the data are presented as the mean + SE of the mean (SEM). The data was analyzed with one-way ANOVA in behavioral results. A post-hoc analysis was performed with Fisher's LSD test when F values reaching significant difference. Statistical analysis was done by SPSS 13.0 (SPSS, Inc., Chicago, IL, USA), P values of < 0.05 are considered statistically significant.

### Results

To investigate the therapeutic potential of RS-D7 in TgM83 mouse model mimicking neuronal alpha-synucleinopathies in related neurodegenerative diseases, the TgM83 mice were treated with RS-D7 (30 mg/kg, P.O.) followed by testing with two motor related behavioral experiments. The experiments included an open field test and a rotarod task performed on two separate days, and together they provide the assessment on the locomotion activity and motor coordination of the animal.

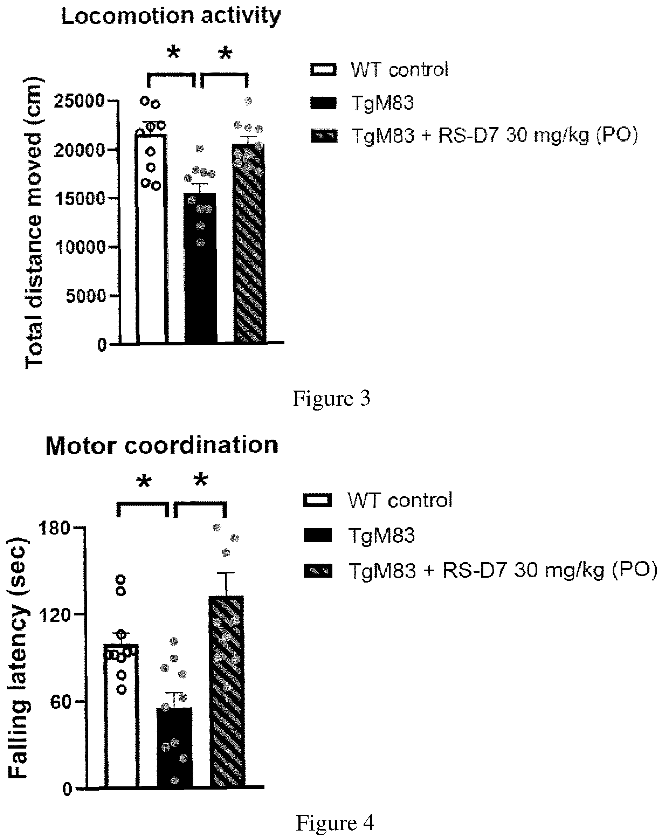

In the open field test, significant difference in the total distance moved was observed among the three groups (WT control: 21,552 ± 1,269, TgM83 mice: 15,472 ± 942, and TgM83 + RS-D7 30 mg/kg (PO): 20,479 ± 730, (cm, mean ± sem); F(2,27) = 10.429, P < 0.05). Fisher's LSD post hoc analysis (Figure 3) showed that compared to the WT control, TgM83 mice exhibited hypolocomotion activity by 6,080 cm moved in the given time of one hour, and oral gavage of 30 mg/kg RS-D7 could significantly rescue the hypolocomotion observed by increasing 5,007 cm of distance moved in the given time (all P < 0.05).

In the Rotarod test, significant difference in the falling latency on the rotarod was observed among the three groups (WT control: 99.5 ± 7.5, TgM83 mice: 55.3 ± 10.4, and TgM83 + RS-D7 30 mg/kg (PO): 132.1 ± 16.1, (seconds, mean ± sem); F(2,27) = 10.502, P < 0.05, Figure 4). Fisher's LSD post hoc analysis (Figure 4) showed that compared to the WT control, TgM83 mice showed motor incoordination demonstrated by the shorter time they took to fall off the rod by 44.2 seconds. Oral gavage of 30 mg/kg RS-D7 significantly ameliorated the motor coordination deficits by prolonging the falling latency to 76.8 seconds (all P < 0.05).

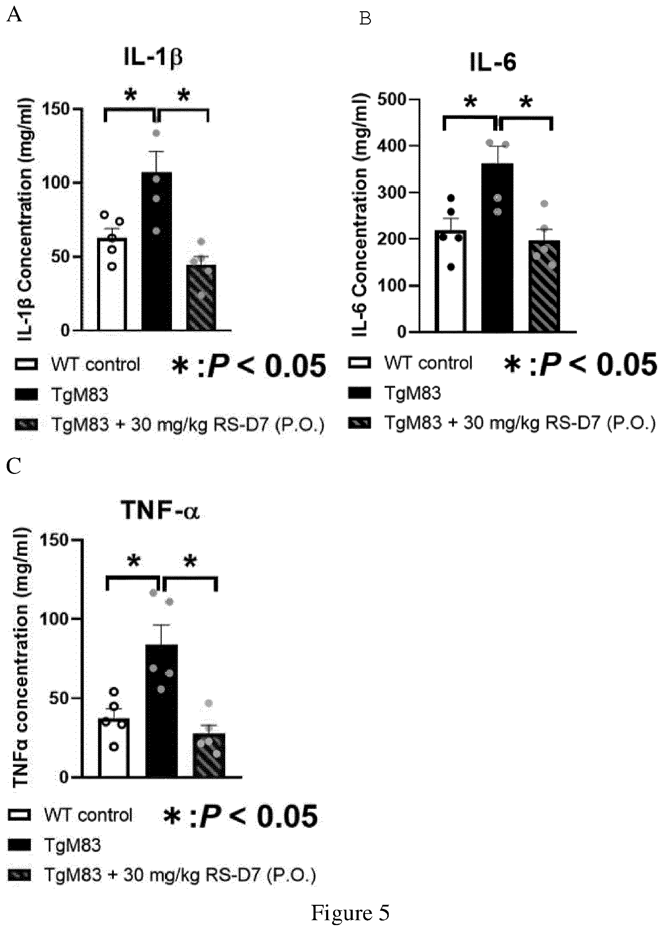

In addition, it is found that RS-D7 treatment inhibited the inflammation cytokines level in the cerebellum of TgM83 mice. To explore the effect of RS-D7 on the inflammatory responses in the TgM83 mice, the inflammation cytokines, including IL-1β, IL-6, and TNF-α, was evaluated in the cerebellum using ELISA. As shown in Figure 5, the significant difference was found in the inflammation cytokines from TgM83 mice in IL-1β (F(3,19) = 11.729, P < 0.05, Figure 5A), IL-6 (F(3,19) = 12.189, P < 0.05, Figure 5B), and TNF-α (F(3,19) = 9.241, P < 0.05, Figure 5C). Fisher's LSD post hoc analysis demonstrated that compared to the WT control group, all IL-1β, IL-6, and TNF-α level were 2-fold overexpressed in TgM83 mice, and Oral gavage of 30 mg/kg RS-D7 significantly inhibit the overexpression of IL-1β, IL-6, and TNF-α in TgM83 mice (all P < 0.05).

As RS-D7 is a new chemical entity, this example investigated the therapeutic effects of RS-D7 in a genetic mouse model of neurodegenerative diseases. A single acute oral gavage of RS-D7 at 30 mg/kg successfully rescued observed motor deficits such as hypolocomotion and motor incoordination in male genetic α-Synuclein aggregated mouse model (TgM83 mice). This example demonstrates the therapeutic effect of RS-D7 and its potential in the treatment of neuronal alpha-synucleinopathies related neurodegenerative diseases.

## Claims

1. A pharmaceutical composition for use in a method for prevention and/or treatment of a disease or disorder mediated by NLRP3 in a subject, comprising administering an effective amount of a compound of Formula (I) or a pharmaceutically acceptable salt, solvate or prodrug as an active ingredient to the subject, 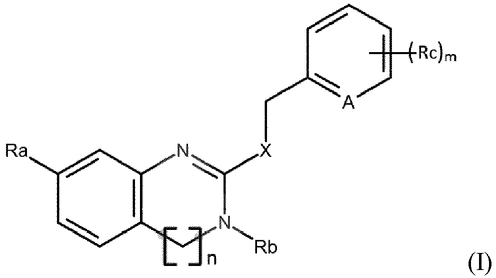
wherein n is 0 or 1,
X is -S-, -S(=O)- or -NRₙ-; wherein
Rₙ is H or 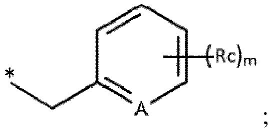
A is -CH, -CR_{c} or N;
Rₐ is -C(=O)ORₐ₁, -ORₐ₂, -O-C(=O)Rₐ₃ or -O-C(=O)-T-ORₐ₄; wherein
Rₐ₁ is H or linear or branched C₁₋₁₅alkyl;
Ra2 is H, linear or branched C₁₋₁₅alkyl, phosphonate, diarylphosphonate or an *O-*protecting group;
Ra3 and Ra4 are independently a protecting group, linear or branched C₁₋₁₅alkyl, linear or branched C₂₋₁₅alkenyl, -T-C₃₋₁₀cycloalkyl, -T-NHRₐ₃ₚ, -T-C₃₋₁₀cycloalkenyl, -T-C6-₁₀aryl, -T-C₅₋₁₀heteroaryl, -T-NH-C(=O)-O-C₁₋₁₀alkyl, -T-adamantyl or -C₁₋₃alkylene-C₆₋₁₀aryl where the alkylene is substituted with -T-NHRₐ₃ₚ;
Rₐ₃ₚ is H or an *N*-protecting group;
Rb is H, linear or branched C₁₋₁₅alkyl, linear or branched C₂₋₁₅alkenyl, C₁₋₃alkoxy-C₁₋₁₅alkyl-, -T'-C₃₋₁₀cycloalkyl, -T'-C₃₋₁₀cycloalkenyl, -T'-C₆₋₁₀ aryl or -T'-C₅₋₁₀heteroaryl;
R_{c} each is independently halogen, linear or branched C₁₋₁₅alkyl, linear or branched C₁₋₁₅alkoxyl, unprotected or protected hydroxyl group, or -C₁₋₁₀alkylene-Y-C₆₋₁₀heteroaryl wherein -Y- is -CH₂-, -NH-, -O- or -S-;
symbol ^{∗} represents the bonding position;
m is an integer from 0 to 4;
-T- is absent, C₁₋₃alkylene or C₂₋₃alkenylene;
-T'- is C₁₋₃alkylene or C₂₋₃alkenylene; and
wherein the heteroaryl contains at least one heteroatom, each heteroatom being independently S, N or O;
wherein the alkyl, alkenyl, alkoxy, cycloalkyl, aryl, heteroaryl, alkylene and alkenylene are each independently unsubstituted or substituted with at least one substituent;
wherein the substituent is each independently a halogen, a protecting group, protected or unprotected amino group, nitro, nitroso, linear or branched C₁₋₁₅ alkyl,or linear or branched C₁₋₁₅ alkoxy or C₃₋₁₀cycloalkyl; and
when R_{b} is H, the tautomers are included,
with the proviso that
when X is -S- or -S(=O)-, Ra is -ORa2 and Ra2 is H or linear or branched C₁₋₁₅alkyl, then A is -CH or -CR_{c};
when X is -S- or -S(=O)- and Ra is -C(=O)ORₐ₁, Rb is linear or branched C₆₋₁₅alkyl, linear or branched C₆₋₁₅alkenyl, C₁₋₃alkoxy-C₁₋₁₅alkyl-, -T'-C₃₋₁₀cycloalkyl, -T'-C₃₋₁₀cycloalkenyl, - T'-C₆₋₁₀ aryl or -T'-C₅₋₁₀heteroaryl;
or a pharmaceutically acceptable salt thereof.

2. The pharmaceutical composition for use of claim 1, wherein the disease or disorder is selected from inflammasome-related diseases or disorders, immune diseases, inflammatory diseases, auto-immune diseases, or auto-inflammatory diseases, liver related diseases or disorders, inflammatory arthritis related disorders, kidney related diseases, neuroinflammation-related diseases, cardiovascular or metabolic diseases or disorders, inflammatory skin diseases, wound healing and scar formation, asthma, sarcoidosis, age- related macular degeneration, or cancer related diseases or disorders; or the disease or disorder is selected from autoinflammatory fever syndromes, chronic liver disease, viral hepatitis, non alcoholic steatohepatitis (NASH), alcoholic steatohepatitis, alcoholic liver disease, gout, pseudogout (chondrocalcinosis), osteoarthritis, rheumatoid arthritis, arthropathy, hyperoxaluria, lupus nephritis, hypertensive nephropathy, hemodialysis related inflammation, multiple sclerosis, brain infection, acute injury, neurodegenerative diseases, Alzheimer's disease (AD), cardiovascular risk reduction (CvRR), hypertension, atherosclerosis, type I and type II diabetes and related complications, peripheral artery disease (PAD), acute heart failure, hidradenitis suppurativa, acne, colon cancer, lung cancer, myeloproliferative neoplasms, leukemias, myelodysplastic syndromes (MDS), myelofibrosis, multiple system atrophy (MSA), or dementia with Lewy bodies; or the disease or disorder is selected from cryopyrin-associated periodic syndrome, nephropathy, or retinopathy; or the disease or disorder is selected from a neurological disorder, symptom domains of schizophrenia and schizoaffective disorder, depression, Tourette Syndrome, Post-traumatic stress disorder (PTSD), Obsessive-compulsive disorder (OCD), analgesics, loss of memory and/or cognition associated with neurodegenerative diseases or loss of neuronal function characteristic of neurodegenerative diseases, mild cognitive impairment (MCI), Alzheimer's disease, Parkinson's disease (PD), schizophrenia, pain, ataxia, convulsion, anxiety, traumatic brain injury (TBI), amyotrophic lateral sclerosis (ALS), multiple sclerosis (MS), fragile X syndrome (FXS), autism, or attention-deficit/hyperactivity disorder (ADHD).

3. The pharmaceutical composition for use of claim 1, wherein n is 0.

4. The pharmaceutical composition for use of claim 1, wherein m is an integer from 0 to 3.

5. The pharmaceutical composition for use of claim 1, wherein:
n is 0;
X is -S(=O);
A is N;
Rₐ is -O-C(=O)Rₐ₃ or -O-C(=O)-T-ORₐ₄; wherein
Ra3 is a linear or branched C₁₋₁₅alkyl, linear or branched C₂₋₁₅alkenyl, -T-C3-₁₀cycloalkyl, -T-NHRₐ₃ₚ, -T-C₃₋₁₀cycloalkenyl, -T-C₆₋₁₀aryl, -T-C₅₋₁₀heteroaryl, -T-NH-C(=O)-O-C₁₋₁₀alkyl, -T-adamantyl or -C₁₋₃alkylene-C₆₋₁₀aryl where the alkylene is substituted with -T-NHRₐ₃ₚ;
Ra4 is a linear or branched C₁₋₁₅alkyl, linear or branched C₂₋₁₅alkenyl, -T-C3-₁₀cycloalkyl, -T-NHRₐ₃ₚ, -T-C₃₋₁₀cycloalkenyl, -T-C₆₋₁₀aryl, -T-C₅₋₁₀heteroaryl, -T-NH-C(=O)-O-C₁₋₁₀alkyl, -T-adamantyl or -C₁₋₃alkylene-C₆₋₁₀aryl where the alkylene is substituted with -T-NHRₐ₃ₚ;
Rₐ₃ₚ is H or an *N*-protecting group selected from Fmoc or Boc;
or a pharmaceutically acceptable salt thereof.

6. The pharmaceutical composition for use of claim 5, wherein:
A is N;
R_{b} is H;
m is 3; and
R_{c} each is independently linear or branched C₁₋₁₅alkyl, linear or branched C₁₋₁₅alkoxyl; or a pharmaceutically acceptable salt.

7. The pharmaceutical composition for use of claim 5, wherein Rₐ is -O-C(=O)Rₐ₃ wherein Ra3 is adamantyl; linear or branched C₁₋₁₀alkyl unsubstituted or substituted by halogen; C₁₋₄alkoxy; -C₆₋₁₀aryl unsubstituted or substituted by C₁₋₁₀ alkyl, nitro, C₁₋₁₅alkoxy or halogen; C₃₋₁₀cycloalkyl; -C₃₋₁₀cycloalkenyl; linear or branched C₂₋₁₀alkenyl; -C₅₋₁₀heteroaryl; -C₁₋₃alkylene-C₃₋₁₀cycloalkyl; C₂₋₃alkenylene-C₆₋₁₀aryl wherein C₆₋₁₀aryl is unsubstituted or substituted by halogen; -O-C(=O)-O-C₁₋₁₀alkyl.

8. The pharmaceutical composition for use of claim 5, wherein Ra is -O-C(=O)Rₐ₃ wherein Ra3 is adamantyl; linear or branched C₁₋₈alkyl unsubstituted or substituted by halogen; C₁₋₄alkoxy; -phenyl unsubstituted or substituted by C₁₋₆ alkyl, nitro, C₁₋₄alkoxy or halogen; C₃₋₆cycloalkyl; - C₃₋₆cycloalkenyl; linear or branched C₂₋₆alkenyl; -C₅₋₆heteroaryl; -C₁₋₃alkylene-C₃₋₆cycloalkyl; C₂₋₃alkenylene-phenyl wherein phenyl is unsubstituted or substituted by halogen; -O-C(=O)-O-C₁₋₄alkyl.

9. The pharmaceutical composition for use of Claim 5, wherein Rₐ is -O-C(=O)-C₁₋₆alkyl, -O-C(=O)-C₁₋₃alkylene-NHFmoc,-O-C(=O)-C₁₋₃alkylene-NHBoc or -O-C(=O)-NH-C(=O)-O-C₁₋₁₀alkyl.

10. The pharmaceutical composition for use of claim 5, wherein R_{c} each is independently halogen, linear or branched C₁₋₆alkyl, linear or branched C₁₋₆alkoxyl, or -C₁₋₁₀alkenylene-Y-C₆₋₁₀heteroaryl; wherein Y is S and C₆₋₁₀heteroaryl is unsubstituted or substituted by C₁₋₁₅alkyl, C₁₋₁₅alkoxy, -OH, -NH2, -NO₂ or halogen.

11. The pharmaceutical composition for use of claim 1, wherein:
X is -S-;
A is N;
Ra is -C(=O)ORₐ₁; wherein Rₐ₁ is H or linear or branched C₁₋₁₅alkyl;
or a pharmaceutically acceptable salt thereof.

12. The pharmaceutical composition for use of claim 11, wherein Ra is -C(=O)OH, or - C(=O)OC₁₋₄alkyl.

13. The pharmaceutical composition for use of claim 1, wherein the compound is selected from the group consisting of:
RS-D7: 5-O-Desmethyl-Omeprazole
Esomeprazole: (S)-(-)-5-Methoxy-2-[(4-methoxy-3,5-dimethylpyridin-2-yl)methylsulfinyl]-3H-benzoimidazole
21122: (2-(((4-methoxy-3,5-dimethylpyridin-2-yl)methyl)sulfinyl)-1H-benzo[d]imidazol-5-yl acetate)
21124: (2-(((4-methoxy-3,5-dimethylpyridin-2-yl)methyl)sulfinyl)-1H-benzo[d]imidazol-5-yl benzoate)
26096: (2-(((4-methoxy-3,5-dimethylpyridin-2-yl)methyl)sulfinyl)-1H-benzo[d]imidazol-5-yl butyrate)
26097: (2-(((4-methoxy-3,5-dimethylpyridin-2-yl)methyl)sulfinyl)-1H-benzo[d]imidazol-5-yl cyclohexanecarboxylate)
26098: (2-(((4-methoxy-3,5-dimethylpyridin-2-yl)methyl)sulfinyl)-1H-benzo[d]imidazol-5-yl 4-butylbenzoate)
21127: (2-(((4-methoxy-3,5-dimethylpyridin-2-yl)methyl)sulfinyl)-1H-benzo[d]imidazol-5-yl 3-methylbenzoate)
27076: 2-(((4-methoxy-3,5-dimethylpyridin-2-yl)methyl)sulfinyl)-1H-benzo[d]imidazol-5-yl hexanoate
27077: 2-(((4-methoxy-3,5-dimethylpyridin-2-yl)methyl)sulfinyl)-1H-benzo[d]imidazol-5-yl isobutyrate
27078: (2-(((4-methoxy-3,5-dimethylpyridin-2-yl)methyl)sulfinyl)-1H-benzo[d]imidazol-5-yl cyclohex-3-ene-1-carboxylate)
27079: 2-(((4-methoxy-3,5-dimethylpyridin-2-yl)methyl)sulfinyl)-1H-benzo[d]imidazol-5-yl cyclohex-3-enecarboxylate
28087: (2-(((4-methoxy-3,5-dimethylpyridin-2-yl)methyl)sulfinyl)-1H-benzo[d]imidazol-5-yl 4-methylbenzoate)
28091: (2-(((4-methoxy-3,5-dimethylpyridin-2-yl)methyl)sulfinyl)-1H-benzo[d]imidazol-5-yl 2-nitrobenzoate)
28092: (2-(((4-methoxy-3,5-dimethylpyridin-2-yl)methyl)sulfinyl)-1H-benzo[d]imidazol-5-yl cyclopropanecarboxylate)
28093: (2-(((4-methoxy-3,5-dimethylpyridin-2-yl)methyl)sulfinyl)-1H-benzo[d]imidazol-5-yl 2-ethylbutanoate)
28094: (2-(((4-methoxy-3,5-dimethylpyridin-2-yl)methyl)sulfinyl)-1H-benzo[d]imidazol-5-yl 2-phenylacetate)
28095: (2-(((4-methoxy-3,5-dimethylpyridin-2-yl)methyl)sulfinyl)-1H-benzo[d]imidazol-5-yl 3,5,5-trimethylhexanoate
28096: (2-(((5-methoxy-4,6-dimethylpyridin-2-yl)methyl)sulfinyl)-1H-benzo[d]imidazol-5-yl 2-ethoxybenzoate)
21123: (2-(((4-methoxy-3,5-dimethylpyridin-2-yl)methyl)sulfinyl)-1H-benzo[d]imidazol-5-yl propionate)
21125: (2-(((4-methoxy-3,5-dimethylpyridin-2-yl)methyl)sulfinyl)-1H-benzo[d]imidazol-5-yl 4-chlorobenzoate)
21126: (2-(((4-methoxy-3,5-dimethylpyridin-2-yl)methyl)sulfinyl)-1H-benzo[d]imidazol-5-yl 3-nitrobenzoate)
21128: 2-(((4-methoxy-3,5-dimethylpyridin-2-yl)methyl)sulfinyl)-1H-benzo[d]imidazol-5-yl heptanoate
21129: (2-(((4-methoxy-3,5-dimethylpyridin-2-yl)methyl)sulfinyl)-1H-benzo[d]imidazol-5-yl 4-fluorobenzoate)
21130: (2-(((4-methoxy-3,5-dimethylpyridin-2-yl)methyl)sulfinyl)-1H-benzo[d]imidazol-5-yl (Z)-2-methylbut-2-enoate)
21131: (2-(((4-methoxy-3,5-dimethylpyridin-2-yl)methyl)sulfinyl)-1H-benzo[d]imidazol-5-yl 2-chloropropanoate)
21132: tert-butyl (2-(((4-methoxy-3,5-dimethylpyridin-2-yl)methyl)sulfinyl)-1H-benzo[d]imidazol-5-yl) carbonate
12124: (2-(((4-methoxy-3,5-dimethylpyridin-2-yl)methyl)sulfinyl)-1H-benzo[d]imidazol-5-yl (Z)-but-2-enoate)
12125: (2-(((4-methoxy-3,5-dimethylpyridin-2-yl)methyl)sulfinyl)-1H-benzo[d]imidazol-5-yl 3-methylbut-2-enoate)
12122: (2-(((4-methoxy-3,5-dimethylpyridin-2-yl)methyl)sulfinyl)-1H-benzo[d]imidazol-5-yl furan-2-carboxylate)
12123: (2-(((4-methoxy-3,5-dimethylpyridin-2-yl)methyl)sulfinyl)-1H-benzo[d]imidazol-5-yl acrylate)
12127: (2-(((4-methoxy-3,5-dimethylpyridin-2-yl)methyl)sulfinyl)-1H-benzo[d]imidazol-5-yl 2-methylbutanoate)
12128: (2-(((4-methoxy-3,5-dimethylpyridin-2-yl)methyl)sulfinyl)-1H-benzo[d]imidazol-5-yl 3-cyclopentylpropanoate)
12129: (2-(((4-methoxy-3,5-dimethylpyridin-2-yl)methyl)sulfinyl)-1H-benzo[d]imidazol-5-yl (E)-3-(2-chlorophenyl)acrylate)
12130: (2-(((4-methoxy-3,5-dimethylpyridin-2-yl)methyl)sulfinyl)-1H-benzo[d]imidazol-5-yl 6-bromohexanoate)
11021: (2-(((4-methoxy-3,5-dimethylpyridin-2-yl)methyl)sulfinyl)-1H-benzo[d]imidazol-5-yl 2-fluorobenzoate)
11020: (2-(((4-methoxy-3,5-dimethylpyridin-2-yl)methyl)sulfinyl)-1H-benzo[d]imidazol-5-yl 4-methoxybenzoate)
11022: (2-(((4-methoxy-3,5-dimethylpyridin-2-yl)methyl)sulfinyl)-1H-benzo[d]imidazol-5-yl (3r,5r,7r)-adamantane-1-carboxylate)
11023: (2-(((4-methoxy-3,5-dimethylpyridin-2-yl)methyl)sulfinyl)-1H-benzo[d]imidazol-5-yl isoxazole-5-carboxylate)
11030: (2-(((4-methoxy-3,5-dimethylpyridin-2-yl)methyl)sulfinyl)-1H-benzo[d]imidazol-5-yl 4-(tert-butyl)benzoate)
11031: (2-(((4-methoxy-3,5-dimethylpyridin-2-yl)methyl)sulfinyl)-1H-benzo[d]imidazol-5-yl 3-chloro-4-fluorobenzoate)
25015: (2-(((4-methoxy-3,5-dimethylpyridin-2-yl)methyl)sulfinyl)-1H-benzo[d]imidazol-5-yl pivalate)
25016: (2-(((4-methoxy-3,5-dimethylpyridin-2-yl)methyl)sulfinyl)-1H-benzo[d]imidazol-5-yl pentanoate)
25017: (2-(((4-methoxy-3,5-dimethylpyridin-2-yl)methyl)sulfinyl)-1H-benzo[d]imidazol-5-yl 4-nitrobenzoate)
25027: (2-(((4-methoxy-3,5-dimethylpyridin-2-yl)methyl)sulfinyl)-1H-benzo[d]imidazol-5-yl cyclobutanecarboxylate)
25028: (2-(((4-methoxy-3,5-dimethylpyridin-2-yl)methyl)sulfinyl)-1H-benzo[d]imidazol-5-yl thiophene-2-carboxylate)
25029: (2-(((4-methoxy-3,5-dimethylpyridin-2-yl)methyl)sulfinyl)-1H-benzo[d]imidazol-5-yl 2-methylbutanoate)
25030: (2-(((4-methoxy-3,5-dimethylpyridin-2-yl)methyl)sulfinyl)-1H-benzo[d]imidazol-5-yl 3,3-dimethylbutanoate)
25031: (2-(((4-methoxy-3,5-dimethylpyridin-2-yl)methyl)sulfinyl)-1H-benzo[d]imidazol-5-yl 2-methoxyacetate) and
25032: (ethyl (2-(((4-methoxy-3,5-dimethylpyridin-2-yl)methyl)sulfinyl)-1H-benzo[d]imidazol-5-yl) carbonate)
or a pharmaceutically acceptable salt thereof.
5. The method of Claim 1, wherein the compound is selected from the group consisting of:
12082: Methyl 1-(2-(cyclohex-1-en-1-yl)ethyl)-2-(((4-methoxy-3,5-dimethylpyridin-2-yl)methyl)thio)-1H-benzo[d]imidazole-5-carboxylate
12083: 1-(2-(cyclohex-1-en-1-yl)ethyl)-2-(((4-methoxy-3,5-dimethylpyridin-2-yl)methyl)thio)- 1H-benzo[d]imidazole-5-carboxylic acid
12084: Methyl 1-(2-(cyclohex-1-en-1-yl)ethyl)-2-(((4-methoxy-3,5-dimethylpyridin-2-yl)methyl) sulfinyl)-1H-benzo[d]imidazole-5-carboxylate
12088: methyl 2-(((4-methoxy-3,5-dimethylpyridin-2-yl)methyl)thio)-1-octyl-1H-benzo [d]imidazole-5-carboxylate
21098: Methyl 2-(((4-methoxy-3,5-dimethylpyridin-2-yl)methyl)amino)-1-propyl-1H-benzo [d]imidazole-5-carboxylate
26065: Methyl 1-(furan-2-ylmethyl)-2-(((4-methoxy-3,5-dimethylpyridin-2-yl)methyl)amino)-1H-benzo[d]imidazole-5-carboxylate
21102: 2-(((4-methoxy-3,5-dimethylpyridin-2-yl)methyl)amino)-1-propyl- 1H-benzo[d]imidazole-5-carboxylic acid
21103: Methyl 2-(((4-methoxy-3,5-dimethylpyridin-2-yl)methyl)amino)-1- (3-methoxypropyl) -1H-benzo[d]imidazole-5-carboxylate
21104: 2-(bis((4-methoxy-3,5-dimethylpyridin-2-yl)methyl)amino)-1- (3-methoxypropyl)-1H-benzo[d]imidazole-5-carboxylic acid
26066: Methyl 2-(((4-methoxy-3,5-dimethylpyridin-2-yl)methyl)amino)-1-pentyl-1H-benzo [d]imidazole-5-carboxylate
21105: 2-(bis((4-methoxy-3,5-dimethylpyridin-2-yl)methyl)amino)-1-(2-(cyclohex-1-en-1-yl)ethyl)-1H-benzo[d]imidazole-5-carboxylic acid
26070: 1-(furan-2-ylmethyl)-2-(((4-methoxy-3,5-dimethylpyridin-2-yl)methyl)amino)-1H-benzo[d]imidazole-5-carboxylic acid
26071: Methyl 2-(((4-methoxy-3,5-dimethylpyridin-2-yl)methyl)amino)-1-(4-methoxybenzyl)-1H-benzo[d]imidazole-5-carboxylate
26072: Methyl 2-(bis((4-methoxy-3,5-dimethylpyridin-2-yl)methyl)amino)-3-(furan-2-ylmethyl)-3,4-dihydroquinazoline-7-carboxylate
21106: Methyl 3-(2-(cyclohex-1-en-1-yl)ethyl)-2-(((4-methoxy-3,5- dimethylpyridin-2-yl)methyl)amino)-3,4-dihydroquinazoline-7-carboxylate
12092: 2-(((4-methoxy-3,5-dimethylpyridin-2-yl)methyl)thio)-1H-benzo[d]imidazol-5-yl (((9H-fluoren-9-yl)methoxy)carbonyl)glycinate
12093: 2-(((4-methoxy-3,5-dimethylpyridin-2-yl)methyl)thio)-1H-benzo[d]imidazol-5-yl (tert-butoxycarbonyl)glycinate
21110: 2-(((4-methoxy-3,5-dimethylpyridin-2-yl)methyl)amino)-3- (3-methoxypropyl)-3,4-dihydroquinazoline-7-carboxylic acid
26076: 2-(((4-methoxy-3,5-dimethylpyridin-2-yl)methyl)amino)-1-pentyl-1H-benzo[d]imidazole-5-carboxylic acid
26077: 2-(((4-methoxy-3,5-dimethylpyridin-2-yl)methyl)amino)-1-(4-methoxybenzyl)-1H-benzo[d]imidazole-5-carboxylic acid
12094: 2-(((4-methoxy-3,5-dimethylpyridin-2-yl)methyl)thio)-1H benzo[d]-imidazole-5-yl (S)-2-((((9H-fluoren-9-yl)methoxy)carbonyl)amino)-2-phenylacetate 13001: 2-(((4-methoxy-3,5-dimethylpyridin-2-yl)methyl)sulfinyl)-1H-benzo[d]imidazol-6-yl 2-((tert-butoxycarbonyl)amino)acetate
13084: 2-(((4-methoxy-3,5-dimethylpyridin-2-yl)methyl)sulfinyl)-1H-benzo[d]imidazol-5-yl diphenyl phosphate
26079: Methyl 3-(furan-2-ylmethyl)-2-(((4-methoxy-3,5-dimethylpyridin-2-yl)methyl)amino)-3,4-dihydroquinazoline-7-carboxylate
26089: 3-(furan-2-ylmethyl)-2-(((4-methoxy-3,5-dimethylpyridin-2-yl)methyl)amino)-3,4-dihydroquinazoline-7-carboxylic acid
26090: 2-(((4-methoxy-3,5-dimethylpyridin-2-yl)methyl)amino)-3-pentyl-3,4-dihydroquinazoline-7-carboxylic acid
26091: Methyl 2-(((4-methoxy-3,5-dimethylpyridin-2-yl)methyl)amino)-3-pentyl-3,4-dihydroquinazoline-7-carboxylate
26092: Methyl 2-(((4-methoxy-3,5-dimethylpyridin-2-yl)methyl)amino)-3-(4-methoxybenzyl)-3,4-dihydroquinazoline-7-carboxylate
21115: 2-(((4-methoxy-3,5-dimethylpyridin-2-yl)methyl)amino)-1- (3-methoxypropyl)-1H-benzo[d]imidazole-5-carboxylic acid
21116: Methyl 1-(2-(cyclohex-1-en-1-yl)ethyl)-2-(((4-methoxy-3,5- dimethylpyridin-2-yl)methyl)amino)-1H-benzo[d]imidazole-5-carboxylate
21117: 1-(2-(cyclohex-1-en-1-yl)ethyl)-2-(((4-methoxy-3,5-dimethylpyridin-2-yl)methyl)amino)-1H-benzo[d]imidazole-5-carboxylic acid
21118: Methyl 2-(((4-methoxy-3,5-dimethylpyridin-2-yl)methyl)amino)-1- phenethyl-1H-benzo[d]imidazole-5-carboxylate
21119: 2-(((4-methoxy-3,5-dimethylpyridin-2-yl)methyl)amino)-1-phenethyl- 1H-benzo[d]imidazole-5-carboxylic acid
21120: 2-(bis((4-methoxy-3,5-dimethylpyridin-2-yl)methyl)amino)-1-propyl- 1H-benzo[d]imidazole-5-carboxylic acid
21121: 2-(bis((4-methoxy-3,5-dimethylpyridin-2-yl)methyl)amino)-1-phenethyl- 1H-benzo[d]imidazole-5-carboxylic acid
22138: 5-methoxy-2-((2-methoxy-3,6-dimethylbenzyl)thio)-1H-benzo[d]imidazole
22139: 5-methoxy-2-((2-methoxy-3,6-dimethylbenzyl)sulfinyl)-1H-benzo[d]imidazole
22140: 2,2'-(((2-methoxy-4-methyl-1,3-phenylene)bis(methylene))bis(sulfanediyl))bis(5-methoxy-1H-benzo[d]imidazole)
21133: 2-((3-(bromomethyl)-2-((tert-butyldimethylsilyl)oxy)-6-methylbenzyl)thio)-5-methoxy-1 H-benzo [d] imidazole and
22141: 2-((2-methoxy-3,6-dimethylbenzyl)thio)-1H-benzo[d]imidazol-5-ol
or a pharmaceutically acceptable salt thereof.

14. The pharmaceutical composition for use of claim 12, wherein R_{c} each is independently halogen, linear or branched C₁₋₆alkyl, linear or branched C₁₋₆alkoxyl, or -C₁₋₁₀alkenylene-Y-C₆₋₁₀heteroaryl; wherein Y is S and C₆₋₁₀heteroaryl is unsubstituted or substituted by C₁₋₁₅alkyl, C2-₁₅alkenyl, C₁₋₁₅alkoxy, -OH, -NH2, -NO₂ or halogen.
